(19) Europäisches Patentamt / European Patent Office / Office européen des brevets

(11) **EP 3 808 159 B1**

(12) **EUROPÄISCHE PATENTSCHRIFT**

(45) Veröffentlichungstag und Bekanntmachung des Hinweises auf die Patenterteilung:
**17.12.2025  Patentblatt 2025/51**

(21) Anmeldenummer: **19732563.2**

(22) Anmeldetag: **13.06.2019**

(51) Internationale Patentklassifikation (IPC):
*H05H 1/24* *(2006.01)*

(52) Gemeinsame Patentklassifikation (CPC):
**H05H 1/2425; H05H 1/2439;** A61N 1/44;
H05H 2240/20; H05H 2242/22

(86) Internationale Anmeldenummer:
**PCT/EP2019/065574**

(87) Internationale Veröffentlichungsnummer:
**WO 2019/238866 (19.12.2019 Gazette 2019/51)**

(54) **VERFAHREN ZUM PRÜFEN EINER ELEKTRODENANORDNUNG ZUR ERZEUGUNG EINES NICHT-THERMISCHEN PLASMAS UND PLASMAQUELLE MIT EINER SOLCHEN ELEKTRODENANORDNUNG, EINGERICHTET ZUR DURCHFÜHRUNG EINES SOLCHEN VERFAHRENS**

METHOD FOR TESTING AN ELECTRODE ARRANGEMENT FOR GENERATING A NON-THERMAL PLASMA, AND PLASMA SOURCE HAVING AN ELECTRODE ARRANGEMENT OF THIS KIND AND CONFIGURED FOR PERFORMING A METHOD OF THIS KIND

PROCÉDÉ DE CONTRÔLE D'UN ENSEMBLE D'ÉLECTRODES POUR LA PRODUCTION D'UN PLASMA NON THERMIQUE ET SOURCE DE PLASMA DOTÉE D'UN TEL ENSEMBLE D'ÉLECTRODES, CONÇUE POUR METTRE EN OEUVRE UN TEL PROCÉDÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.06.2018  DE 102018209730**

(43) Veröffentlichungstag der Anmeldung:
**21.04.2021  Patentblatt 2021/16**

(73) Patentinhaber:
• **terraplasma GmbH**
  **85748 Garching (DE)**
• **terraplasma medical GmbH**
  **85748 Garching (DE)**

(72) Erfinder:
• **ZIMMERMANN, Julia**
  **81925 München (DE)**
• **LINNER, Michael**
  **81549 München (DE)**
• **CANTZLER, Sylvia**
  **85560 Ebersberg (DE)**
• **MORFILL, Gregor**
  **81925 München (DE)**
• **WEILEMANN, Hannes**
  **81925 München (DE)**
• **CANTZLER, Maximilian**
  **85560 Ebersberg (DE)**

(74) Vertreter: **Kordel, Mattias et al**
**Gleiss Große Schrell und Partner mbB**
**Patentanwälte Rechtsanwälte**
**Leitzstrasse 45**
**70469 Stuttgart (DE)**

(56) Entgegenhaltungen:
EP-A1- 1 693 014          WO-A2-2017/147625
CN-A- 107 787 108         JP-A- 2001 259 409
US-A1- 2014 207 053

# EP 3 808 159 B1

## Beschreibung

Die Erfindung betrifft ein Verfahren zum Prüfen einer Elektrodenanordnung zur Erzeugung eines nicht-thermischen Plasmas, sowie eine Plasmaquelle mit einer solchen Elektrodenanordnung, wobei die Plasmaquelle eingerichtet ist zur Durchführung eines solchen Verfahrens.

Nicht-thermische Plasmen werden in vielfältigen Anwendungsfällen zur Reduzierung einer Anzahl von pathogenen Keimen oder zur Eliminierung solcher Keime eingesetzt, beispielsweise auf dem Gebiet der Wundbehandlung, der Behandlung von Hauterkrankungen, der Lebensmittelhygiene, der Herstellung von Wasser zur intravenösen Injektion, der Trinkwasseraufbereitung, der Dekontamination, Desinfektion oder Sterilisation von Gegenständen, insbesondere von medizinischen Geräten und/oder im militärischen Bereich, im zivilen Bereich, im Bereich der Luft- und Raumfahrt, insbesondere der Oberflächenbehandlung, ganz besonders der Oberflächen-Sterilisation oder -Desinfektion, der Inaktivierung von Allergenen, der Saatgutbehandlung, des Pflanzenschutzes, der Geruchsreduktion, beispielsweise beim Auffrischen von Textilien oder Textilprodukten, insbesondere von Kleidung oder Matratzen, der Luftreinigung und -überwachung, und vielerlei dergleichen mehr. Nicht-thermische Plasmen werden zur Wundbehandlung und zur Behandlung von Hauterkrankungen auch deswegen eingesetzt, weil sie über ihre keimlastreduzierende Wirkung hinaus heilungsfördernde Wirkungen aufweisen. Zahlreiche dieser Anwendungen sind kritisch in dem Sinne, dass für den Anwender selbst oder für denjenigen, an dem oder für den die Anwendung durchgeführt wird, beispielsweise einen Patienten oder einen Verbraucher von derart behandeltem Trinkwasser, einem Verwender von Saatgut oder dergleichen, nicht unerhebliche Gefahren drohen können, wenn das nicht-thermische Plasma bei der Anwendung nicht oder nicht in ausreichendem Umfang erzeugt und appliziert wird. Auch kann aus Sicherheitsgründen eine Limitierung einer Maximaldosis bestimmter Spezies eingehalten werden müssen. Bei einer flächigen oder geometrisch linearen Plasmaerzeugung ist es auch wichtig, Informationen darüber zu erlangen, ob das Plasma entlang einer Fläche oder Linie, auf der es erzeugt werden soll, gleichmäßig erzeugt wird, da ansonsten die Gefahr droht, dass bestimmte Flächen- oder Linienbereiche in verringertem Maß oder gar nicht behandelt werden, während andere Bereiche gegebenenfalls in überhöhtem Maß mit dem Plasma beaufschlagt werden.

Es besteht daher Bedarf an einem Verfahren, mit dem zuverlässig festgestellt werden kann, ob eine Elektrodenanordnung zur Erzeugung eines nicht-thermischen Plasmas eine bestimmungsgemäße Funktion aufweist.

EP1693014 A1 offenbart eine Vorrichtung und ein Verfahren zur Kalibrierung der Abgabe von thermischer Energie bei einem Plasmagerät zur Behandlung von Gewebeoberflächen.

Der Erfindung liegt die Aufgabe zugrunde, ein Verfahren zum Prüfen einer Elektrodenanordnung zur Erzeugung eines nicht-thermischen Plasmas zu schaffen, welches dazu beiträgt, die genannten Nachteile zu vermeiden, wobei es insbesondere ermöglichen soll, die Elektrodenanordnung auf eine bestimmungsgemäße Funktionsfähigkeit zu überprüfen. Weiterhin liegt der Erfindung die Aufgabe zugrunde, eine Plasmaquelle zu schaffen, welche eine Elektrodenanordnung zur Erzeugung eines nicht-thermischen Plasmas aufweist, wobei die Plasmaquelle eingerichtet ist zur Durchführung des zuvor genannten Verfahrens.

Die Aufgabe wird gelöst, indem die Gegenstände der unabhängigen Ansprüche geschaffen werden. Vorteilhafte Ausgestaltungen ergeben sich aus den Unteransprüchen.

Die Aufgabe wird insbesondere gelöst, indem ein Verfahren zum Prüfen einer Elektrodenanordnung zur Erzeugung eines nicht-thermischen Plasmas geschaffen wird, welches folgende Schritte aufweist: Es wird wenigstens ein Leistungs-Parameter bestimmt, der für eine Plasma-Leistung der Elektrodenanordnung charakteristisch ist. Der Leistungs-Parameter wird insbesondere im Betrieb, aber vor der Anwendung der Elektrodenanordnung bestimmt; er ist insbesondere charakteristisch für eine momentane Plasma-Leistung der Elektrodenanordnung während deren Betrieb aber vor der Anwendung. Der wenigstens eine bestimmte Leistungs-Parameter wird mit wenigstens einem vorbestimmten Soll-Parameterwert verglichen, und es wird aus diesem Vergleich ein Vergleichsergebnis erhalten. Anhand des Vergleichsergebnisses wird eine Funktionsfähigkeit der Elektrodenanordnung beurteilt. Mithilfe des hier vorgeschlagenen Verfahrens ist es insbesondere initial bei einer Inbetriebnahme der Elektrodenanordnung möglich, deren Funktionsfähigkeit sicher und genau zu bestimmen und so insbesondere mit einer mangelhaften, reduzierten oder nicht vorhandenen Funktionsfähigkeit der Elektrodenanordnung verbundene Gefahren für einen Verwender der Elektrodenanordnung oder für Dritte zu vermeiden. Vorzugsweise wird in Abhängigkeit von dem Vergleichsergebnis wenigstens eine Aktion ausgewählt. Auf diese Weise ist es möglich, auf das Vergleichsergebnis und damit auch auf die festgestellte Funktionsfähigkeit der Elektrodenanordnung zu reagieren und eine hierauf angepasste Aktion auszuwählen.

Unter einem nicht-thermischen Plasma wird ein Plasma verstanden, bei welchem eine die Verteilung der kinetischen Energie von Elektronen des Plasmas beschreibende Temperatur, die auch als Elektronen-Temperatur bezeichnet wird, nicht identisch und insbesondere sehr viel höher ist als eine die Verteilung der kinetischen Energie der von dem Plasma umfassten Ionen, insbesondere Atomionen und/oder Molekülionen, beschreibende Temperatur, die auch als Ionen-Temperatur bezeichnet wird. Dabei ist die Elektronen-Temperatur sehr viel höher als die Ionen-Temperatur, wobei die Ionen-Temperatur besonders im Bereich von 25 °C bis höchstens 100 °C gewählt werden kann. Ein solches Plasma wird aufgrund der vergleichsweise niedrigen Ionen-Temperatur auch als kaltes Plasma bezeichnet.

Als Plasma wird hier ein Materiezustand bezeichnet, bei dem geladene Teilchen mit positiven und negativen Ladungen in Gasphase nebeneinander vorliegen, wobei sich über ein bestimmtes Volumen gemittelt eine neutrale elektrische Ladung für das betrachtete Volumen ergibt. Das Plasma umfasst außerdem vorzugsweise nicht-geladene Atome und/oder Moleküle, die sich in elektronisch-, vibratorisch- und/oder rotatorisch angeregten Zuständen befinden, und die auch als angeregte Teilchen bezeichnet werden, und/oder freie Radikale, insbesondere also nicht-geladene reaktive Atome und/oder Moleküle, die auch als reaktive Teilchen oder reaktive Spezies bezeichnet werden.

Unter einer Elektrodenanordnung wird hier eine Anordnung elektrisch leitfähiger Elektroden relativ zueinander verstanden, die eingerichtet ist, um ein nicht-thermisches Plasma zu erzeugen, wenn an die Elektrodenanordnung eine Spannung, insbesondere eine Wechselspannung, angelegt wird. Unter einer Plasmaquelle wird eine Einrichtung verstanden, die über die Elektrodenanordnung im engeren Sinne hinaus, also die Anordnung elektrisch leitender Elektroden relativ zueinander, Mittel zur Beaufschlagung der Elektroden mit elektrischer Leistung, insbesondere eine Spannungsquelle, und bevorzugt eine Steuereinrichtung zur Beeinflussung, Vorgabe, Messung, Auswertung, Steuerung und/oder Regelung der an die Elektroden angelegten Stromgrößen, mithin insbesondere einer Spannung und/oder einer Stromstärke, aufweist.

In dem Verfahren gemäß der Erfindung, weist die Elektrodenanordnung bevorzugt eine erste Elektrode und eine zweite Elektrode auf, die durch ein Dielektrikum voneinander beabstandet sind, sodass Plasmaentladungen, insbesondere Oberflächenmikroentladungen, an einer der beiden Elektroden erzeugt werden können, wenn eine Spannung, insbesondere eine Wechselspannung, an die Elektroden angelegt wird. Die Elektrodenanordnung ist somit eingerichtet, um ein nicht-thermisches Plasma unabhängig von einer zu der Elektrodenanordnung externen Oberfläche zu erzeugen. Insbesondere ist die Elektrodenanordnung als SMD-Elektrodenanordnung (Surface Micro Discharge) ausgebildet. Insbesondere bedarf es keiner als Gegenelektrode geschalteten, zu behandelnden Oberfläche. Die beiden Elektroden der Elektrodenanordnung stehen vorzugsweise in physischem Kontakt mit dem Dielektrikum, wobei sie in das Dielektrikum eingebettet, oder aber auf dem Dielektrikum angeordnet sein können, beispielsweise durch Aufdampfen, Siebdruck, physikalische oder chemische Gasphasenabscheidung, durch Auflegen oder Anpressen, Aufkleben, oder in anderer geeigneter Weise, wobei sie in diesem Fall dicht und insbesondere ohne Luftspalt an dem Dielektrikum angeordnet sind. Somit sind insbesondere beide Elektroden der Elektrodenanordnung auf einer gemeinsamen Seite einer zu behandelnden Oberfläche angeordnet. Insbesondere ist die zu behandelnde Oberfläche nicht zwischen den beiden Elektroden der Elektrodenanordnung angeordnet.

Die Elektrodenanordnung ist insbesondere eingerichtet zur Erzeugung eines nicht-thermischen Plasmas in Luft, insbesondere in Umgebungsluft. Es wird also bevorzugt kein spezielles Gas zur Erzeugung des nicht-thermischen Plasmas verwendet, insbesondere wird kein solches spezielles Gas, insbesondere kein Trägergas, der Elektrodenanordnung zugeführt.

Insbesondere zur Prüfung einer solchen Elektrodenanordnung ist das hier vorgeschlagene Verfahren geeignet. Insbesondere bei einer solchen Elektrodenanordnung ist auch die Plasma-Leistung direkt abhängig von einer Fläche oder Linie, entlang der oder auf welcher das Plasma erzeugt wird, wobei etwaige Ungleichmäßigkeiten in der Plasmaerzeugung und/oder Flächenteile und/oder Linienbereiche, auf oder in denen beispielsweise aufgrund von Verschmutzungen kein Plasma erzeugt wird, direkt die Plasma-Leistung reduzieren. Somit kann anhand des Vergleichs des Leistungs-Parameters mit dem wenigstens einen vorbestimmten Soll-Parameterwert auch festgestellt werden, ob das Plasma gleichmäßig erzeugt wird.

Unter einer Plasma-Leistung der Elektrodenanordnung wird derjenige Teil der von der Elektrodenanordnung aufgenommenen elektrischen Leistung verstanden, der direkt zur Erzeugung des nicht-thermischen Plasmas eingesetzt wird und insbesondere direkt mit einer Erzeugungsrate für von dem Plasma umfasste reaktive Teilchen in Zusammenhang steht. Wird als Leistungs-Parameter ein Parameter erfasst, der für diese Plasma-Leistung charakteristisch ist, kann in besonders sicherer und zuverlässiger Weise auf die Funktionsfähigkeit der Elektrodenanordnung geschlossen werden, weil der Leistungs-Parameter in diesem Fall unmittelbar Auskunft über die Plasma-Erzeugung durch die Elektrodenanordnung gibt.

Unter einem Beurteilen der Funktionsfähigkeit der Elektrodenanordnung wird insbesondere verstanden, dass eine Aussage über die Funktionsfähigkeit der Elektrodenanordnung abgeleitet wird, sei dies indirekt durch Auswahl einer bestimmten Aktion und/oder direkt durch Ausgeben einer die Funktionsfähigkeit der Elektrodenanordnung beschreibenden oder bezeichnenden Meldung. Die Funktionsfähigkeit kann dabei im Sinne einer einfachen, binären Feststellung beurteilt werden, ob nämlich die Elektrodenanordnung funktionsfähig ist oder nicht, es ist aber auch möglich, dass die Funktionsfähigkeit der Elektrodenanordnung in komplexerer Weise beurteilt wird, insbesondere in Hinblick auf eine Ermittlung der momentanen Plasma-Leistung und gegebenenfalls der Auswahl einer Aktion in Abhängigkeit von der ermittelten momentanen Plasma-Leistung.

Bevorzugt wird im Rahmen des Verfahrens die Plasma-Leistung als Leistungs-Parameter oder anhand des wenigstens einen Leistungs-Parameters erfasst.

Gemäß einer Weiterbildung der Erfindung ist vorgesehen, dass die Aktion ausgewählt ist aus einer Gruppe, bestehend aus einer Ausgabe eines "In-Ordnung"-Signals, einer Ausgabe eines "Handlungsbedarf"-Signals, einer Ausgabe eines "Nicht-In-Ordnung"-Signals, einer Mitteilung der - insbesondere momentanen - Plasma-Leistung an einen Betreiber der Elektrodenanordnung, einer Anpassung einer Betriebsdauer oder Behandlungsdauer an das Vergleichsergebnis, einem Beenden eines Betriebs der Elektrodenanordnung vor Start der Anwendung, und einem Fortsetzen des Betriebs der Elektrodenanordnung zur Anwendung ohne weitere Maßnahme, insbesondere ohne Ausgabe eines Signals oder einer Mitteilung. Ein "In-Ordnung"-Signal wird auch als Grünsignal bezeichnet, ein als "Handlungsbedarf"-Signal wird im Folgenden auch als Gelb-Alarm bezeichnet, und ein "Nicht-In-Ordnung"-Signal wird im Folgenden auch als Rot-Alarm bezeichnet. Dabei zeigt ein Grünsignal an, dass die Elektrodenanordnung bestimmungsgemäß arbeitet.

Das Grünsignal kann insbesondere ausgegeben werden, wenn der wenigstens eine Leistungs-Parameter von dem vorbestimmten Soll-Parameterwert um weniger als einen ersten vorbestimmten Grenzwert abweicht, beispielsweise um weniger als 15 %. Ein Gelb-Alarm informiert einen Betreiber der Elektrodenanordnung darüber, dass die Elektrodenanordnung überprüft werden sollte, wobei gegebenenfalls weitere Schritte, beispielsweise ein Reinigen der Elektrodenanordnung, ein Säubern von Kontakten, oder andere derartige Maßnahmen, erforderlich sein könnten. Ein solcher Gelb-Alarm wird bevorzugt ausgegeben, wenn die Abweichung des wenigstens einen Leistungs-Parameters von dem wenigstens einen vorbestimmten Soll-Parameterwert größer ist als der erste vorbestimmte Grenzwert, wobei sie kleiner ist als ein zweiter vorbestimmter Grenzwert, wobei der zweite vorbestimmte Grenzwert größer ist als der erste vorbestimmte Grenzwert. Der zweite vorbestimmte Grenzwert kann beispielsweise einer Abweichung um 30 % von dem vorbestimmten Soll-Parameterwert entsprechen. Der Gelb-Alarm kann auch ausgegeben werden, wenn die Abweichung des wenigstens einen Leistungs-Parameters von dem wenigstens einen vorbestimmten Soll-Parameterwert gleich dem ersten vorbestimmten Grenzwert ist. Ein Rot-Alarm kann insbesondere ausgegeben werden, wenn ein weiteres Betreiben der Elektrodenanordnung aufgrund mangelnder Funktionsfähigkeit nicht mehr sinnvoll oder entweder für die Elektrodenanordnung selbst, für den späteren Nutzer eines mit dem Plasma behandelten Guts (z.B. in der Trinkwasseraufbereitung), den Betreiber, oder für eine mit der Elektrodenanordnung behandelte Person gefährlich ist. Der Rot-Alarm kann insbesondere ausgegeben werden, wenn der wenigstens eine Leistungs-Parameter von dem wenigstens einen vorbestimmten Soll-Parameterwert um den zweiten vorbestimmten Grenzwert oder um mehr als den zweiten vorbestimmten Grenzwert abweicht.

Der wenigstens eine vorbestimmte Soll-Parameterwert kann gemäß einer Ausführungsform des Verfahrens ein Sollwert sein, wobei in diesem Fall der Leistungs-Parameter mit dem - insbesondere genau einen - Sollwert verglichen wird und die Funktionsfähigkeit der Elektrodenanordnung anhand des Vergleichsergebnisses beurteilt wird. Dabei ist insbesondere eine Abweichung von dem Sollwert sowohl nach oben als auch nach unten zumindest bei Überschreiten bestimmter relativ zu dem Sollwert definierter Grenzwerte ein Anzeichen für eine mangelnde Funktionsfähigkeit der Elektrodenanordnung.

Gemäß einer anderen Ausführungsform des Verfahrens ist es möglich, dass der wenigstens eine vorbestimmte Soll-Parameterwert ein Minimalwert ist. In diesem Fall wird der Leistungs-Parameter mit dem Minimalwert in der Weise verglichen, dass geprüft wird, ob der Leistungs-Parameter größer oder kleiner als der Minimalwert ist. Dabei ist die Elektrodenanordnung funktionsfähig, wenn der Leistungs-Parameter größer oder gleich dem Minimalwert ist, wobei die Elektrodenanordnung nicht funktionsfähig ist, wenn der Leistungs-Parameter kleiner als der Minimalwert ist. Dabei kann ebenfalls ein Bereich für einen Gelb-Alarm definiert werden, wobei dieser sich dann ausgehend von dem Minimalwert bis zu einem vorbestimmten Grenzwert erstreckt, der um einen vorbestimmten Betrag oder einen vorbestimmten Faktor kleiner ist als der Minimalwert. Der Bereich für den Rot-Alarm erstreckt sich dann ausgehend von dem vorbestimmten Grenzwert hin zu kleineren Werten, wobei der Bereich für den Gelb-Alarm zwischen dem vorbestimmten Grenzwert und dem Minimalwert angeordnet ist. Der Bereich für das Grün-Signal liegt in diesem Fall oberhalb des Minimalwerts.

In entsprechender - lediglich umgekehrter - Weise kann bei einer weiteren Ausführungsform des Verfahrens der Soll-Parameterwert als Maximalwert definiert sein. Die Elektrodenanordnung ist dann nicht funktionsfähig, wenn der Leistungs-Parameter Werte oberhalb des Maximalwerts annimmt, wobei die Elektrodenanordnung funktionsfähig ist, wenn der Leistungs-Parameter Werte unterhalb oder bis zu dem Maximalwert annimmt. Der Bereich des Grün-Signals erstreckt sich dann von kleineren Werten, insbesondere von null her bis zu dem Maximalwert, wobei der Bereich des Gelb-Alarms sich ausgehend von dem Maximalwert bis zu einem vorbestimmten Grenzwert erstreckt, der um einen vorbestimmten Betrag oder einen vorbestimmten Faktor größer ist als der vorbestimmte Grenzwert. Der Bereich des Rot-Alarms erstreckt sich dann ausgehend von dem vorbestimmten Grenzwert zu höheren Werten.

Bei einer weiteren Ausführungsform des Verfahrens ist es möglich, dass zwei vorbestimmte Soll-Parameterwerte vorgesehen werden, mit denen der wenigstens eine Leistungs-Parameter verglichen wird. Die beiden vorbestimmten Soll-Parameterwerte definieren dabei ein Werteband oder Grenzen eines Wertebereichs, wobei die Elektrodenanordnung innerhalb dieses Wertebands oder Wertebereichs als funktionsfähig beurteilt wird. Insbesondere ist dabei ein erster vorbestimmter Soll-Parameterwert als Minimalwert des Wertebands oder Wertebereichs definiert, wobei ein zweiter, größerer Soll-Parameterwert als Maximalwert des Wertebands oder Wertebereichs definiert ist. Die Bereiche für den Gelb-Alarm sind dann jeweils dem Maximalwert einerseits und dem Minimalwert andererseits in dem Sinne zugeordnet, wie dies zuvor für den Minimalwert und den Maximalwert erläutert wurde.

Es ist alternativ auch möglich, dass ein Bereich für einen Gelb-Alarm nicht - wie oben erläutert - ausgehend von dem vorbestimmten Soll-Parameterwert sich in Richtung des Bereichs für den Rot-Alarm erstreckt, sondern dass er sich vielmehr in den Bereich des Grün-Signals erstreckt. In diesem Fall kann beispielsweise der dem Minimalwert zugeordnete vorbestimmte Grenzwert größer sein als der Minimalwert, wobei der dem Maximalwert zugeordnete vorbestimmte Grenzwert kleiner sein kann als der Maximalwert. Alternativ ist es auch möglich, einen Gelb-Alarmbereich so zu definieren, dass er den vorbestimmten Soll-Parameterwert einschließt, vorzugsweise symmetrisch umfasst.

Der wenigstens eine vorbestimmte Soll-Parameterwert wird vorzugsweise abhängig von einem gewünschten Betriebsmodus der Elektrodenanordnung gewählt, insbesondere abhängig von einer gewünschten Plasmachemie, insbesondere einer gewünschten Konzentration bestimmter aktiver Spezies in dem Plasma. Beispielsweise ist es möglich, dass verschiedene, insbesondere einen zulässigen Wertebereich oder ein zulässiges Werteband begrenzende Soll-Parameterwerte vorgegeben werden einerseits für den Fall, dass das erzeugte, nicht-thermische Plasma im Wesentlichen Sauerstoff-Spezies, beispielsweise Ozon, umfassen soll (Sauerstoffmodus), oder dass das nicht-thermische Plasma im Wesentlichen Stickstoff-Spezies, insbesondere Stickoxide, umfassen soll (Stickstoffmodus). Auch ist es möglich, einen Zwischenbereich zwischen diesen Betriebsmodi zu wählen. Dabei hängt die Plasmachemie stark von der gewählten Plasma-Leistung ab und kann durch diese somit vorgegeben werden. Insoweit muss auch die Funktionsfähigkeit der Elektrodenanordnung mit Blick auf die Plasma-Leistung in Abhängigkeit von dem gewählten Betriebsmodus untersucht werden.

Die hier beschriebenen Signale können beispielsweise als Licht-Signale ausgegeben werden. Insbesondere ist es möglich, dass das Grünsignal als grün leuchtendes Licht, der Gelb-Alarm als gelb leuchtendes Licht, und der Rot-Alarm als rot leuchtendes Licht ausgegeben wird. Zur Ausgabe der Lichtsignale können insbesondere Leuchtdioden verwendet werden.

Die Signale und/oder Mitteilungen können aber alternativ oder zusätzlich auch in Textform, insbesondere in einem Display, als akustische Signale oder Mitteilungen, durch Vibration, oder in anderer geeigneter Weise ausgegeben werden.

Eine Mitteilung der insbesondere momentanen Plasma-Leistung an den Betreiber ermöglicht es diesem, ein Behandlungsergebnis der Elektrodenanordnung bei einer bestimmten Behandlungsdauer abzuschätzen, und gegebenenfalls die Behandlungsdauer an die momentane Plasma-Leistung noch vor dem Beginn der Anwendung anzupassen. Weist beispielsweise die Elektrodenanordnung eine im Vergleich zu einer Nenn-Plasma-Leistung reduzierte momentane Plasma-Leistung auf, kann die Behandlungsdauer durch den Betreiber in geeigneter Weise verlängert werden, um eine bestimmte Plasma-Dosis zu applizieren. Eine solche Anpassung der Behandlungsdauer kann aber bevorzugt auch automatisch erfolgen, insbesondere in Abhängigkeit von dem Vergleichsergebnis. Der Betreiber wird dann vorzugsweise über die automatisch geänderte Behandlungsdauer informiert, oder der Betreiber ist angehalten, die Elektrodenanordnung bis zu deren selbstständigen Beendigen des Betriebs zu betreiben, wobei dann die geänderte Behandlungsdauer quasi automatisch berücksichtigt wird. Die Behandlungsdauer entspricht dabei bevorzugt einer Betriebsdauer der Elektrodenanordnung, da diese vorzugsweise nur während einer tatsächlich durchgeführten Behandlung auch betrieben wird. Eine Behandlung beginnt dann insbesondere mit der Inbetriebnahme der Elektrodenanordnung und endet mit der Beendigung des Betriebs der Elektrodenanordnung.

Der Betrieb der Elektrodenanordnung kann insbesondere beendet oder gesperrt werden, wenn ein Weiterbetreiben derselben nicht mehr sinnvoll oder für die Elektrodenanordnung selbst, den Betreiber oder eine mit der Elektrodenanordnung behandelte Person gefährlich ist. Insbesondere kann der Betrieb der Elektrodenanordnung gleichzeitig mit der Ausgabe eines Rot-Alarms beendet oder gesperrt werden.

Wird eine ungeminderte Funktionsfähigkeit der Elektrodenanordnung festgestellt, wird vorzugsweise deren weiterer Betrieb zugelassen oder fortgesetzt. Insbesondere kann das Zulassen oder Fortsetzen des Betriebs der Elektrodenanordnung zugleich mit der Ausgabe eines Grünsignals erfolgen, insbesondere wenn das Prüfverfahren bei Inbetriebnahme der Elektrodenanordnung durchgeführt wird.

Gemäß einer Weiterbildung der Erfindung ist vorgesehen, dass das Verfahren unmittelbar nach einer Inbetriebnahme der Elektrodenanordnung - aber vorzugsweise noch vor deren Anwendung - durchgeführt wird. Insbesondere ist es möglich, dass das Verfahren unmittelbar nach jeder Inbetriebnahme der Elektrodenanordnung - stets neu, vorzugsweise automatisch - durchgeführt wird. Auf diese Weise kann die Elektrodenanordnung direkt bei Inbetriebnahme überprüft werden, wobei bevorzugt dem Betreiber der Elektrodenanordnung eine Rückmeldung ausgegeben wird, ob die Elektrodenanordnung funktionsfähig ist. Auf diese Weise kann stets vor dem eigentlichen Einsatz der Elektrodenanordnung, insbesondere vor einer Behandlung einer Oberfläche, einer Flüssigkeit, eines Schüttguts, oder einer Person mit der Elektrodenanordnung, festgestellt werden, ob die Elektrodenanordnung funktionsfähig ist, wobei gegebenenfalls der eigentliche Einsatz der Elektrodenanordnung nicht erfolgt und diese vielmehr überprüft, gereinigt oder einer Reparatur zugeführt wird. Dies hat zum einen den Vorteil, dass der Betreiber frühzeitig über Probleme der Elektrodenanordnung informiert wird, sodass eine Fehlbehandlung oder gegebenenfalls unbemerkt nicht stattfindende Behandlung vermieden wird, und wobei direkt Maßnahmen eingeleitet werden können, um die Funktionsfähigkeit der Elektrodenanordnung zu erhalten oder sicherzustellen. Auch für gegebenenfalls anzufertigende Behandlungsprotokolle ist es vorteilhaft, wenn unmittelbar bei Inbetriebnahme der Elektrodenanordnung vermerkt werden kann, ob oder dass diese funktionsfähig ist.

Vorzugsweise wird das Verfahren ausschließlich vor einer Anwendung, insbesondere nicht während einer Anwendung der Elektrodenanordnung durchgeführt. Es dient also insbesondere als Vorab-Funktionstest vor dem eigentlichen Gebrauch.

In einer nicht zur Erfindung gehörenden Ausgestaltung ist vorgesehen, dass der wenigstens eine vorbestimmte Soll-Parameterwert konstant vorgegeben ist. Dies kann insbesondere dann der Fall sein, wenn äußere Bedingungen, unter denen die Elektrodenanordnung geprüft wird, stets zumindest näherungsweise identisch sind, und/oder wenn der wenigstens eine vorbestimmte Soll-Parameterwert hinreichend unempfindlich auf variierende äußere Bedingungen ist. Es wird insoweit optional ein Leistungs-Parameter verwendet, der höchstens in geringem Umfang mit äußeren Bedingungen der Elektrodenanordnung variiert. Weiter optional wird der Leistungs-Parameter so gewählt, dass seine Beziehung zur tatsächlichen Plasma-Leistung der Elektrodenanordnung nur in geringem Maß, insbesondere nicht, von solchen äußeren Bedingungen, insbesondere einer Temperatur, Luftfeuchtigkeit, Alterungseffekten wie Korrosion, Oxidation, Ablagerungen und dergleichen abhängt. Auf diese Weise kann gewährleistet werden, dass der Leistungs-Parameter in jedem Fall nur von der Plasma-Leistung der Elektrodenanordnung abhängt und somit stets ausschließlich für die Plasma-Leistung der Elektrodenanordnung charakteristisch ist.

Gemäß der Erfindung ist der wenigstens eine vorbestimmte Soll-Parameterwert in Abhängigkeit von wenigstens einem Einsatzparameter der Elektrodenanordnung hinterlegt, wobei er abhängig von dem wenigstens einen Einsatzparameter abgerufen oder aktiviert werden kann. Der wenigstens eine vorbestimmte Soll-Parameterwert ist in Abhängigkeit von dem wenigstens einen Einsatzparameter in einem Kennfeld hinterlegt, aus dem er abhängig von dem wenigstens einen Einsatzparameter ausgelesen wird. Dies ist insbesondere sinnvoll, wenn sich die Einsatzbedingungen der Elektrodenanordnung und damit ein Wert des wenigstens einen Einsatzparameters im Laufe der Zeit ändern können. Der wenigstens eine Einsatzparameter ist ausgewählt aus einer Gruppe, bestehend aus einer Umgebungstemperatur der Elektrodenanordnung und einer relativen Luftfeuchte in einer Umgebung der Elektrodenanordnung. Dies ist insbesondere relevant bei der Behandlung feuchter Oberflächen oder feuchter oder nasser Umgebungen, beispielsweise für die Wundbehandlung oder Wasseraufbereitung. Dabei kann es bevorzugt genügen, für den wenigstens einen vorbestimmten Soll-Parameterwert in Abhängigkeit von der relativen Luftfeuchte zwei verschiedene Werte zu hinterlegen, beispielsweise einen ersten Wert für eine relative Luftfeuchte von 80 % oder mehr als 80 %, und einen zweiten Wert für eine relative Luftfeuchte von weniger als 80 %.

Es ist möglich, dass der wenigstens eine Einsatzparameter durch die Elektrodenanordnung oder eine die Elektrodenanordnung aufweisende Plasmaquelle gemessen wird. Auf diese Weise können stets direkt aktuelle und genaue Werte des Einsatzparameters erhalten werden. Alternativ oder zusätzlich ist es möglich, dass der wenigstens eine Einsatzparameter durch die Elektrodenanordnung oder die Plasmaquelle erhalten wird, insbesondere von einer externen Quelle; beispielsweise ist es möglich, dass der wenigstens eine Einsatzparameter von einem Dienstgeber oder Computer heruntergeladen, aus einem Netzwerk bezogen, oder durch den Betreiber der Elektrodenanordnung über eine geeignete Schnittstelle eingegeben wird.

Das Plasma wird durch die Elektrodenanordnung bevorzugt in Umgebungsluft erzeugt. Der Elektrodenanordnung wird also kein separates Trägergas für die Plasmaerzeugung zugeführt. Daher ist der Betrieb der Elektrodenanordnung auch in besonderer Weise durch die Umgebungstemperatur und/oder relative Luftfeuchte in der Umgebung der Elektrodenanordnung beeinflusst.

Die Elektrodenanordnung wird bevorzugt mit Wechselspannung betrieben, insbesondere mit einer Frequenz vorzugsweise von mindestens 2 kHz bis höchstens 100 kHz. Die Elektrodenanordnung wird bevorzugt bei einer Spannung von einigen Kilovolt betrieben, wobei die Spannung vorzugsweise von mindestens 1 kV$_{pp}$ bis höchstens 5 kV$_{pp}$, bevorzugt zu 3,5 kV$_{pp}$ gewählt wird.

Gemäß einer Weiterbildung der Erfindung ist vorgesehen, dass die Elektrodenanordnung für die Ermittlung des Leistungs-Parameters geheizt wird. Besonders bevorzugt wird hierzu die Elektrodenanordnung auf eine Temperatur von mindestens 50 °C erwärmt. Auf diese Weise ist es möglich, gegebenenfalls auf der Elektrodenanordnung oberflächlich abgelagerte Feuchtigkeit zu entfernen, die ansonsten die Messung beeinträchtigen kann.

Die Plasma-Leistung kann insbesondere auf verschiedene Weise bestimmt werden:
Eine bevorzugte Möglichkeit ist die Fourier- (oder Power Spektrum-) Analyse, wobei nur die Leistung im hochfrequenten Teil des Spektrums bestimmt wird. Da die Plasmaentladungen viele kleine "Spikes" erzeugen (praktisch wie Delta-Funktionen) ist die Plasma-Leistung im Hochfrequenzbereich messbar.

In einer anderen bevorzugten Messmethode wird die Plasma-Leistung durch die Fläche einer Lissajous-Figur beschrieben, die erzeugt wird durch eine Phasenraumdarstellung einer Ansteuerspannung, die definiert ist als diejenige Spannung, die mittels einer Spannungsquelle an die Elektrodenanordnung zur Plasmaerzeugung angelegt wird, gegen eine Plasmaspannung, die definiert ist als die Spannung, die tatsächlich über der Elektrodenanordnung in deren Betrieb anliegt, wobei demnach die Ansteuerspannung die unmodifizierte Betriebsspannung der Plasmaquelle ist und die Plasmaspannung die durch Plasmaentladungen modifizierte/verformte, relativ zur Ansteuerspannung phasenverschobene Spannung, die über der Elektrodenanordnung abfällt. Hier werden bevorzugt nicht die einzelnen Mikroentladungen im Spannungsverlauf berücksichtigt, sondern eine geeignete Mittelung. Die Phasenraumdarstellung erzeugt eine geschlossene Kurve um eine Einschlussfläche. Diese Einschlussfläche enthält Information über die Verformung der Ansteuerspannung durch die Mikroentladungen sowie die Phasenverschiebung zwischen Ansteuerspannung und Plasmaspannung und stellt somit ein Maß für die Plasma-Leistung dar.

**In** der Praxis ist es aus verschiedenen Gründen nicht immer möglich, diese Phasenraumdarstellung zu nutzen und/oder die Spannungsverläufe direkt zu messen. Dann bedeutet Ansteuerspannung: angelegte Hochspannung oder Spannung, die der angelegten Hochspannung in Form, Phase und Amplitude entspricht; und anstelle der Plasmaspannung wird eine Proxyspannung gemessen, die über einer mit der Elektrodenanordnung in Reihe geschalteten elektronischen Proxystruktur abfällt - insbesondere auch als "Proxy-Messung" bezeichnet -, wobei die Proxyspannung die beiden durch die Mikroentladungen (die die eigentliche Plasma-Leistung beinhalten) hervorgerufenen Effekte (Verformung und Phasenverschiebung) abbildet. Die eingeschlossene Fläche dieser "Proxy-Messung" oder auch die Proxyspannung für sich genommen beschreibt ebenfalls die Plasma-Leistung.

Es gibt verschiedene Möglichkeiten eine solche "Proxy-Messung" durchzuführen, die die Plasma-Leistung abbildet:

Ad 1. Die Phasenraumkurve der Ansteuerspannung gegen die Proxyspannung wird aufgetragen, und das Integral der so aufgespannten Fläche wird gebildet.

Ad 2. Bei einem vorgegebenen Zeitpunkt in der Sinuskurve der Ansteuerspannung wird die Proxyspannung gemessen. Optimal ist die Positionierung dieses Zeitpunktes der Ansteuerspannung derart gewählt, dass die maximale Breite und/oder Höhe der Lissajous-Figur getroffen wird. Diese Position liegt optimal in dem Bereich der größten zeitlichen Gradienten und/oder Phasendifferenz zwischen der Ansteuerspannung und der Proxyspannung.

Ein leicht zu definierender Punkt für diese Messung ist der Nulldurchgang der Ansteuerspannung. Die Proxyspannung an dieser Stelle ist nah der maximalen Breite oder Höhe der Lissajous-Figur. Die so erfasste Proxyspannung ist ein leicht messbarer Parameter, der die Plasma-Leistung abbildet. Hierfür bedarf es noch der geeigneten Wahl eines Proportionalitätsfaktors, der insbesondere durch Vergleich mit der eingeschlossenen Fläche der Lissajous-Figur bestimmt werden kann.

Wegen der "Diskretisierung" der Messung kann bei einer solch "singulären" Messung zufällig eine Mikroentladung getroffen werden oder nicht. Deshalb werden bevorzugt genügend viele Messungen - bevorzugt 256 Messungen - gemittelt, um ein zuverlässiges Ergebnis für die Plasma-Leistung zu erhalten.

Gemäß einer Weiterbildung der Erfindung ist demnach vorgesehen, dass der wenigstens eine Leistungs-Parameter an einer zu der Elektrodenanordnung in Reihe geschalteten elektronischen Proxystruktur, insbesondere einer elektronischen Proxystruktur der die Elektrodenanordnung aufweisenden Plasmaquelle, - insbesondere als Proxy-Messung - erfasst wird. Dies ermöglicht eine einfache, insbesondere auch bei einem kleinen, tragbaren, handhaltbaren Gerät durchführbare Messung des Leistungs-Parameters, die gleichwohl charakteristisch für die Plasma-Leistung der Elektrodenanordnung ist.

Unter einer elektronischen Proxystruktur wird hier insbesondere ein elektronisches Bauteil oder eine Mehrzahl miteinander elektrisch mittelbar oder unmittelbar verbundener und miteinander zusammenwirkender elektronischer Bauteile verstanden, die insbesondere geeignet ist, um daran eine Stellvertretermessung zur Ermittlung des wenigstens einen Leistungs-Parameters und letztlich der Plasma-Leistung vorzunehmen.

Gemäß einer Weiterbildung der Erfindung ist vorgesehen, dass als elektronische Proxystruktur eine Kapazität verwendet wird. Dabei wird unter einer Kapazität allgemein eine elektronische Struktur verstanden, die sich zumindest auch kapazitiv, vorzugsweise im Wesentlichen kapazitiv, vorzugsweise ausschließlich kapazitiv, verhält. Besonders bevorzugt wird als elektronische Proxystruktur wenigstens ein Kondensator oder eine Kondensator-Anordnung, besonders bevorzugt genau ein Kondensator, verwendet. Es hat sich herausgestellt, dass die Verwendung einer Kapazität als elektronische Proxystruktur im Rahmen des hier vorgeschlagenen Verfahrens eine besonders verlässliche Aussage über die tatsächliche Plasma-Leistung der Elektrodenanordnung erlaubt.

Die Kapazität der elektronischen Proxystruktur - im Folgenden als Proxykapazität bezeichnet - wird bevorzugt größer, insbesondere sehr viel größer, vorzugsweise um einen Faktor von mindestens 500 bis höchstens 2000, vorzugsweise von mindestens 750 bis höchstens 1500, vorzugsweise von 1000, größer gewählt, als es der Kapazität der Elektrodenanordnung im Plasmabetrieb - im Folgenden als Anordnungskapazität bezeichnet - entspricht.

Die Proxyspannung $V_{proxy}$ verhält sich zu der Plasmaspannung $V_{plasma}$ in folgender Weise:

$$V_{proxy} = \frac{C_a}{C_a + C_p} V_{plasma}, \qquad (1)$$

wobei $C_p$ die Proxykapazität und $C_a$ die Anordnungskapazität sind.

Dies sei an einem bevorzugten Ausführungsbeispiel näher erläutert:

(Anfang des bevorzugten Ausführungsbeispiels.) Die Anordnungskapazität ist vorzugsweise proportional zu einer Gesamt-Kantenlänge L (Summe aller Kantenlängen) einer strukturierten Elektrode der Elektrodenanordnung, an deren Kanten die Plasma-Erzeugung erfolgt, und ergibt sich dann als

$$C_a = c_L \cdot L \qquad\qquad (2)$$

mit dem Proportionalitätsfaktor $c_L$.

Die Anordnungskapazität beträgt beispielsweise 109 pF, die Plasmaspannung betrage 3,5 $kV_{pp}$ (Spitze zu Spitze). Weiterhin ist die Gesamt-Kantenlänge L gleich 72 cm. Damit ist $c_L = C_a/L = 1{,}51$ - solch ein Wert ist typisch für SMD-Elektrodenanordnungen, wobei $c_L$ im Bereich $1 < c_L < 2$ liegt.

Aus messtechnischen Gründen möchte man einen Wert für die Proxyspannung in etwa von 3 bis 5 $V_{pp}$ haben. Daraus ergibt sich eine Skalierung (wobei $C_p \gg C_a$):

$$C_p = \frac{c_L \cdot L}{V_{proxy}} V_{plasma}. \qquad\qquad (3)$$

Die Größe der Plasmaspannung ist anhand der Ansteuerspannung bekannt (typisch einige kV), die gewünschte Proxyspannung ebenfalls. Für eine im Wesentlichen durch die Gesamt-Kantenlänge L vorgegebene Elektrodenkonfiguration und die Elektrodenbauart (z.B. SMD - das definiert $c_L$) kann $C_p$ bestimmt werden.

Für eine bevorzugte Elektrodenanordnung ergibt sich aus der Gleichung (3) ein Richtwert für die Proxykapazität von $C_p = 100$ nF (mit $V_{proxy} = 3{,}5$ $V_{pp}$ und $V_{plasma} = 3{,}5$ $kV_{pp}$. (Ende des bevorzugten Ausführungsbeispiels.)

Gemäß einer Weiterbildung der Erfindung ist vorgesehen, dass als der wenigstens eine Leistungs-Parameter wenigstens ein Wert der Proxyspannung zu einem bestimmten Phasenwinkel der Ansteuerspannung, insbesondere bei einem Nulldurchgang der Ansteuerspannung, gemessen wird. Vorzugsweise wird als der wenigstens eine Leistungs-Parameter ein Mittelwert PM der Proxyspannung bei dem bestimmten Phasenwinkel der Ansteuerspannung, gemittelt über eine Mehrzahl, insbesondere eine Vielzahl, von Perioden der Ansteuerspannung ermittelt:

$$PM = \frac{1}{n}\sum_{i=1}^{n} V_{proxy,i}(\varphi), \qquad\qquad (4)$$

wobei in Gleichung (4) $V_{proxy,i}(\varphi)$ der Wert der Proxyspannung bei dem festgehaltenen Phasenwinkel $\varphi$ - insbesondere bei dem Nulldurchgang - der Ansteuerspannung in der Periode i ist, und wobei n eine Anzahl der Perioden der Ansteuerspannung ist, über welche die Mittelung erfolgt. Dabei ist gemäß einer bevorzugten Ausgestaltung n = 256; gemäß einer anderen bevorzugten Ausgestaltung kann n einen anderen oder größeren Wert annehmen. Bei n = 256 ergibt sich für eine Frequenz der Ansteuerspannung von x kHz eine Berechnung des Mittelwerts der in jeder Periode fortlaufend einmal gemessenen Proxyspannung alle 1/(4x) Sekunden, wenn alle Messungen konsekutiv in aufeinanderfolgenden Perioden stattfinden. Insbesondere bei hohen Frequenzen ist auch eine Messung nur in bestimmten Perioden möglich (z.B. jede zweite oder dritte Periode, usw.), oder man misst alle 256 Perioden hintereinander und lässt dann eine Lücke von einer bestimmten Anzahl von Perioden. Für die Ermittlung der Plasma-Dosis muss die entsprechende Vorgehensweise natürlich berücksichtigt werden.

In einer Steuereinrichtung zur Ansteuerung der Elektrodenanordnung ist vorzugsweise eine Zuordnung des Leistungs-Parameters zu der tatsächlichen Plasma-Leistung hinterlegt, vorzugsweise als einfacher Faktor oder als komplexere, bevorzugt zumindest injektive, vorzugsweise bijektive Funktion, die einem gemessenen Wert des Leistungs-Parameters eindeutig eine tatsächliche Plasma-Leistung zuordnet.

Gemäß einer Weiterbildung der Erfindung ist vorgesehen, dass der Leistungs-Parameter mit einem ersten, oberen Soll-Parameterwert und mit einem zweiten, unteren Soll-Parameterwert verglichen wird. Dabei ist der erste, obere Soll-Parameterwert größer als der zweite, untere Soll-Parameterwert. Die wenigstens eine Aktion wird abhängig davon gewählt, ob der Leistungs-Parameterwert in einen durch den ersten Soll-Parameterwert und den zweiten Soll-Parameterwert begrenzten Soll-Parameterbereich fällt. Der erste Soll-Parameterwert und der zweite Soll-Parameterwert spannen also einen Soll-Parameterbereich auf, in den der Leistungs-Parameter bestimmungsgemäß fallen soll; dies bedeutet, dass die Elektrodenanordnung ordnungsgemäß funktioniert, wenn der Leistungs-Parameter in den Soll-Parameterbereich fällt. Ist der Leistungs-Parameter dagegen kleiner als der zweite, untere Soll-Parameterwert oder größer als der erste, obere Soll-Parameterwert, funktioniert die Elektrodenanordnung nicht ordnungsgemäß und ist entweder nicht oder nur eingeschränkt einsetzbar. Dabei kann die wenigstens eine Aktion insbesondere auch abhängig davon gewählt werden, wie weit der Leistungs-Parameter von dem ersten, oberen Soll-Parameterwert oder von dem zweiten, unteren Soll-Parameterwert - außerhalb des Soll-Parameterbereichs - beabstandet ist. Hierbei ist es insbesondere möglich, einen Bereich für einen Gelb-Alarm und einen Bereich für einen Rot-Alarm durch entsprechende weitere Grenzwerte zur separieren.

Der erste, obere Soll-Parameterwert berücksichtigt dabei eine obere Leistungsgrenze für die Plasmaerzeugung, wobei diese obere Leistungsgrenze beispielsweise durch Erosion eines Dielektrikums der Elektrodenanordnung, Deposition auf dem Dielektrikum, Kriechstrombildung oder andere, ähnliche, die Leistungsaufnahme der Elektrodenanordnung erhöhende Effekte überschritten werden kann. Der untere, zweite Soll-Parameterwert berücksichtigt eine untere Leistungsgrenze der Elektrodenanordnung, die beispielsweise durch Verschmutzung, Deposition und/oder Erosion leitender Bestandteile einer Elektrode der Elektrodenanordnung unterschritten werden kann, oder durch andere, ähnliche Effekte, welche die Leistungsaufnahme der Elektrodenanordnung verringern.

Bevorzugt wird jede Elektrodenanordnung im Rahmen einer Anfangsprüfung charakterisiert, wobei die ersten und zweiten Soll-Parameterwerte individuell für die jeweilige Elektrodenanordnung und Anwendungsaufgabe (z.B. Sauerstoff, Stickstoff oder Intermediärer Modus) festgelegt und vorzugsweise in einer der Elektrodenanordnung zugeordneten elektronischen Speichereinrichtung, beispielsweise einem RFID-Chip oder dergleichen, hinterlegt werden. Auf diese Weise können intra-individuelle Fertigungsvariationen erfasst und ein möglichst genauer, bestimmungsgemäßer Funktionsbereich für die individuelle Elektrodenanordnung und Anwendungsaufgabe vorgegeben werden. Diese individuellen Schwellenwerte können dann für jede Elektrodenanordnung, auch bei einem Austausch der Elektrodenanordnung in einer existierenden Plasmaquelle, übertragen werden, insbesondere indem die Speichereinrichtung ausgelesen wird. Die Speichereinrichtung, beispielsweise der RFID-Chip oder ein anderer Datenträger, ist vorzugsweise mit der Elektrodenanordnung mitnehmbar verbunden und wird gemeinsam mit dieser an der Plasmaquelle angeordnet und/oder ausgetauscht.

Gemäß einer Weiterbildung der Erfindung ist vorgesehen, dass die Elektrodenanordnung vor der Bestimmung des mindestens einen Leistungs-Parameters für eine vorbestimmte Zeitdauer betrieben wird. Auf diese Weise kann gewährleistet werden, dass sich konstante Betriebsbedingungen und/oder ein Gleichgewicht für den Betrieb der Elektrodenanordnung eingestellt hat/haben, so dass der Leistungs-Parameter korrekt erfasst wird.

Gemäß einer Weiterbildung der Erfindung ist vorgesehen, dass das Vergleichsergebnis und/oder der wenigstens eine Leistungs-Parameter für einen späteren Abruf in einer elektronischen Speichereinrichtung protokolliert wird/-werden. Die elektronische Speichereinrichtung kann direkt in die Steuereinrichtung der Plasmaquelle integriert, aber auch extern hierzu vorgesehen sein. Insbesondere ist es möglich, dass die Protokollierung in einem externen Dienstgeber erfolgt, der mit der Steuereinrichtung über eine kabelgebundene oder kabellose Datenverbindung, beispielsweise WLAN und/oder Bluetooth, wirkverbunden ist. Besonders bevorzugt wird/werden das Vergleichsergebnis und/oder der wenigstens eine Leistungs-Parameter automatisiert protokolliert, und/oder besonders bevorzugt mit wenigstens einem Metadatum verknüpft, beispielsweise einem Zeitstempel, einer Angabe über einen Ort des Einsatzes der Elektrodenanordnung, eine Angabe über einen Einsatzzweck oder eine Einsatzart der Elektrodenanordnung, Angaben über bestimmte Parameter des Betriebs der Elektrodenanordnung, oder dergleichen. Auf diese Weise kann quasi ein Logbuch für den Betrieb der Elektrodenanordnung erstellt werden, sodass deren Funktionsfähigkeit und Einsatzbereitschaft, oder auch allgemein deren Betrieb mit der Zeit nachvollzogen werden kann/können.

Vorzugsweise ist es auch möglich, die Elektrodenanordnung über eine kabelgebundene oder kabellose Wirkverbindung, insbesondere eine Funkverbindung, vorzugsweise WLAN und/oder Bluetooth, fernzuüberwachen, auszulesen und/oder zu steuern, besonders bevorzugt per Internetzugriff und/oder über eine Smartphone-App.

Gemäß einer Weiterbildung der Erfindung ist vorgesehen, dass eine Elektrodenanordnung geprüft wird, die eingerichtet ist zur Erzeugung von Oberflächenmikroentladungen in Umgebungsluft. Gerade eine solche Elektrode kann mit dem hier vorgeschlagenen Verfahren überprüft werden. Die Plasma-Erzeugung erfolgt dabei flächig oder entlang einer Linie, insbesondere an Kanten einer strukturierten Elektrode der Elektrodenanordnung, direkt in Umgebungsluft. Vorzugsweise ist der Elektrodenanordnung ein Abstandshalter zugeordnet, um einen bestimmten Abstand zu einer zu behandelnden Oberfläche zu gewährleisten. Der Abstandshalter ist bevorzugt so ausgestaltet, dass er mit der zu behandelnden Oberfläche im Betrieb der Elektrodenanordnung ein Volumen einschließt, sodass das Plasma durch die Elektrodenanordnung in einem geschlossenen Volumen erzeugt wird.

Gemäß einer Weiterbildung der Erfindung ist vorgesehen, dass eine Elektrodenanordnung geprüft wird, die eine erste, insbesondere flächige Elektrode sowie eine zweite, vorzugsweise flächige Elektrode aufweist. Die Elektrodenanordnung weist außerdem ein Dielektrikum auf, durch welches die erste Elektrode und die zweite Elektrode voneinander beabstandet sind, wobei die erste Elektrode und die zweite Elektrode jeweils - in Stapelrichtung des Stapels aus den Elektroden und dem Dielektrikum gesehen auf gegenüberliegenden Seiten des Dielektrikums - in mechanischem Kontakt mit dem Dielektrikum sind. Sie können dabei insbesondere auf gegenüberliegenden Oberflächen des Dielektrikums angeordnet oder zumindest bereichsweise in das Dielektrikum eingebettet sein. Besonders bevorzugt ist die erste Elektrode dicht anliegend an einer ersten Seite des Dielektrikums angeordnet, wobei die zweite Elektrode dicht anliegend an einer zweiten, der ersten Seite gegenüberliegenden Seite des Dielektrikums angeordnet ist, beispielsweise durch Aufdampfen, Siebdruck, physikalische oder chemische Gasphasenabscheidung, durch Auflegen oder Anpressen, Aufkleben, oder in anderer geeigneter Weise.

Auf diese Weise wird eine Elektrodenanordnung geschaffen, welche geeignet ist, an einer der beiden Elektroden auf einer Seite des Dielektrikums, insbesondere an Kanten dieser Elektrode, Oberflächenmikroentladungen zu erzeugen und so ein nicht-thermisches Plasma zu generieren, ohne dass eine behandelte Oberfläche zwischen den Elektroden und/oder einer Elektrode und dem Dielektrikum angeordnet werden muss, und weiter ohne dass die zu behandelnde Oberfläche selbst als Gegenelektrode geschaltet werden muss. Des Weiteren ist es möglich, das nicht-thermische Plasma zumindest weitgehend gleichmäßig an der Oberfläche zu erzeugen, an welcher die Oberflächenmikroentladungen gezündet werden, sodass über diese Oberfläche gleichmäßige und konstante Bedingungen und Plasma-Parameter erreicht werden können.

Bevorzugt ist die zweite Elektrode gegen die zweite Seite des Dielektrikums gedrängt oder gepresst, sie liegt also bevorzugt insbesondere unter Vorspannung oder Anpresskraft an der zweiten Seite des Dielektrikums an. Dies ermöglicht eine dichte und stabile Anordnung der zweiten Elektrode an der zweiten Seite des Dielektrikums ohne einen Luftspalt, was günstig für die Effizienz und die Plasmaerzeugungsrate der Plasmaquelle ist. Zugleich kann die Elektrodenanordnung auf einfache Weise gefertigt werden, insbesondere da die zweite Elektrode separat von dem Dielektrikum gefertigt werden kann, und dann nur noch auf dieses aufgelegt und unter Vorspannung gesetzt oder angepresst werden muss. Auch ist die zweite Elektrode sehr einfach austauschbar.

Es ist möglich, dass auch die erste Elektrode gegen die erste Seite des Dielektrikums gedrängt oder gepresst ist, insbesondere unter Vorspannung oder Anpresskraft. Besonders bevorzugt ist allerdings die erste Elektrode auf das Dielektrikum beschichtet, insbesondere aufgedampft.

Die zweite Elektrode weist bevorzugt eine periodische Struktur aus einer Mehrzahl identischer Strukturelemente auf, und/oder die zweite Elektrode weist wenigstens ein Strukturelement mit wenigstens einer von Kanten begrenzten Ausnehmung auf. Die zweite Elektrode ist demnach als strukturierte Elektrode ausgebildet, die Kanten aufweist, an denen Oberflächenmikroentladungen gezündet werden können.

Die die Ausnehmung begrenzenden Kanten weisen bevorzugt innerhalb jeder Ausnehmung eine Kantenlänge von mindestens 0,5 mm bis höchstens 10 mm, vorzugsweise von mindestens 1 mm bis höchstens 8 mm, vorzugsweise von mindestens 2 mm bis höchstens 7 mm, vorzugsweise von 5 mm zueinander auf.

Zusätzlich oder alternativ ist bevorzugt vorgesehen, dass die zweite Elektrode eine Mehrzahl von Strukturelementen aufweist, wobei die einzelnen Strukturelemente einen Abstand von mindestens 0,5 mm bis höchstens 10 mm, vorzugsweise von mindestens 1 mm bis höchstens 8 mm, vorzugsweise von mindestens 2 mm bis höchstens 7 mm, vorzugsweise von 5 mm, zueinander aufweisen.

Die erste Elektrode ist bevorzugt mit einer Isolierschicht und/oder Vergussmasse versehen.

Gemäß einer Weiterbildung der Erfindung ist vorgesehen, dass im Betrieb der Elektrodenanordnung an die erste Elektrode eine Hochspannung, insbesondere eine Wechselspannung, vorzugsweise mit einer Amplitude von mindestens 1 kV$_{pp}$ bis höchstens 5 kV$_{pp}$, und/oder mit einer Frequenz von mindestens 2 kHz bis höchstens 100 kHz, angelegt wird. Die Frequenz wird bevorzugt insbesondere in Abhängigkeit der Eigenschaften einer verwendeten Hochspannungsquelle gewählt. Die zweite Elektrode wird bevorzugt auf Masse gelegt oder geerdet.

Im Betrieb der Elektrodenanordnung zur Behandlung einer Oberfläche ist die erste Elektrode vorzugsweise der zu behandelnden Oberfläche abgewandt, wobei die zweite Elektrode der zu behandelnden Oberfläche zugewandt ist. Dabei erstreckt sich die Stapelrichtung des Stapels der Elektrodenanordnung aus der ersten Elektrode, der zweiten Elektrode und dem Dielektrikum schräg oder quer, bevorzugt senkrecht zu der zu behandelnden Oberfläche. Damit ist die elektrische Sicherheit mit Blick auf die zu behandelnde Oberfläche besonders hoch, da diese bei entsprechender bestimmungsgemäßer Anwendung höchstens mit der zweiten, auf Masse gelegten oder geerdeten Elektrode in Kontakt kommen kann.

Zugleich ist die zweite Elektrode diejenige, an welcher die Oberflächenmikroentladungen gezündet und damit das Plasma erzeugt wird, wobei dieses ungehindert auf die zu behandelnde Oberfläche einwirken kann.

Das hier vorgeschlagene Verfahren ist grundsätzlich für eine Vielzahl verschiedener, insbesondere flächiger oder linearer Elektrodenanordnungen durchführbar. Insbesondere ist es für Elektrodenanordnungen durchführbar, die nach dem Prinzip der dielektrischen Barriereentlandung (Dielectric Barrier Discharge - DBD), dem Prinzip der Oberflächenmikroentladung (Surface Micro Discharge - SMD), und/oder als beschichtete SMD-Elektrode, aufgebaut sind. Das Verfahren ist besonders geeignet für Elektrodenanordnungen, die kapazitiv gekoppelt sind.

Besonders bevorzugt wird das Verfahren allerdings durchgeführt für eine Elektrodenanordnung, die im Folgenden in Zusammenhang mit der hier vorgeschlagenen Plasmaquelle näher beschrieben wird:
Die Aufgabe wird insbesondere gelöst, indem eine Plasmaquelle mit einer Elektrodenanordnung zum Erzeugen eines nicht-thermischen Plasmas geschaffen wird, wobei die Elektrodenanordnung eine erste Elektrode, eine zweite Elektrode, und ein Dielektrikum aufweist, durch das die erste Elektrode und die zweite Elektrode voneinander beabstandet sind. Dabei ist die erste Elektrode an einer ersten Seite des Dielektrikums angeordnet, wobei die zweite Elektrode an einer zweiten, der ersten Seite gegenüberliegenden Seite des Dielektrikums angeordnet ist. Die Plasmaquelle weist außerdem eine Steuereinrichtung auf, die eingerichtet ist zur Ansteuerung der Elektrodenanordnung. Die Steuereinrichtung ist außerdem eingerichtet zur Durchführung eines erfindungsgemäßen Verfahrens. In Zusammenhang mit der Plasmaquelle verwirklichen sich insbesondere die Vorteile, die bereits in Zusammenhang mit dem Verfahren erläutert wurden.

Die erste Elektrode ist vorzugsweise dicht an der ersten Seite des Dielektrikums angeordnet. Alternativ oder zusätzlich ist bevorzugt die zweite Elektrode dicht an der zweiten Seite des Dielektrikums angeordnet. Es ist auch möglich, dass zumindest eine Elektrode, ausgewählt aus der ersten Elektrode und der zweiten Elektrode, in das Dielektrikum eingebettet ist. Auch können beide Elektroden in das Dielektrikum eingebettet sein. Es ist aber auch möglich, dass zumindest eine Elektrode, ausgewählt aus der ersten Elektrode und der zweiten Elektrode, gegen die hier zugeordnete Seite des Dielektrikums gedrängt ist. Besonders bevorzugt ist die zweite Elektrode gegen die zweite Seite des Dielektrikums gedrängt.

Gemäß einer Ausgestaltung der Plasmaquelle ist es möglich, dass die zweite Elektrode ein Material aufweist, das ausgewählt ist aus einer Gruppe, bestehend aus Edelstahl, Titan, Wolfram, einem elektrisch leitfähigen Kunststoff, und einem Leitkleber. Die hier für die zweite Elektrode angegebenen Materialien weisen eine gute elektrische Leitfähigkeit auf, wobei sie zugleich hart gegen Sputtering, somit dauerfest und langzeitstabil, und resistent gegen Oxidation, insbesondere bei Einwirkung von Ozon sind. Somit eignen sich diese Materialien in besonderer Weise für eine dauerhafte Anwendung gerade auch im medizinischen Bereich und insbesondere bei Ozonerzeugung, wobei sie zusätzlich kostengünstig bereitgestellt werden können.

Dass die zweite Elektrode gegen die zweite Seite des Dielektrikums gedrängt ist, bedeutet insbesondere, dass sie in diesem Fall nicht in das Dielektrikum eingebettet und nicht stoffschlüssig mit dem Dielektrikum verbunden ist. Vielmehr ist die zweite Elektrode vorzugsweise unter mechanischer Vorspannung an der zweiten Seite des Dielektrikums gehalten, insbesondere an die zweite Seite des Dielektrikums angedrückt, oder auf die zweite Seite des Dielektrikums aufgepresst. Auf diese Weise kann die Elektrodenanordnung sehr einfach und kostengünstig hergestellt werden, wobei zugleich aufgrund der Vorspannkraft, welche die zweite Elektrode gegen das Dielektrikum drängt, Luft aus einem Spalt zwischen der zweiten Elektrode und dem Dielektrikum wirksam verdrängt werden kann, sodass eine möglichst dichte Anlage der zweiten Elektrode an der zweiten Seite des Dielektrikums erreicht wird. Bei Beschädigung ist die zweite Elektrode sehr einfach austauschbar, da sie ohne weiteres und insbesondere zerstörungsfrei von dem Dielektrikum gelöst werden kann, insbesondere indem die mechanische Vorspannung beseitigt wird. Es ist insbesondere möglich, dass die zweite Elektrode durch ein Vorspannelement oder ein Anpresselement gegen die zweite Seite des Dielektrikums gedrängt ist.

Besonders bevorzugt ist die gesamte Elektrodenanordnung durch Vorspannkräfte aneinandergehalten, wobei die Elektrodenanordnung insbesondere zusammengedrückt oder -gepresst sein kann.

Es ist bevorzugt auch möglich, dass die erste Elektrode auf das Dielektrikum beschichtet, insbesondere aufgedampft ist. Alternativ oder zusätzlich ist es möglich, dass die zweite Elektrode auf das Dielektrikum beschichtet, insbesondere aufgedampft ist.

Die Elektrodenanordnung ist insbesondere eingerichtet zur Erzeugung von Oberflächen-Mikroentladungen.

Wird an die beiden Elektroden, das heißt die erste Elektrode und die zweite Elektrode, eine Potentialdifferenz, insbesondere eine Wechselspannung angelegt, bilden sich an einer aktiven Oberfläche der Elektrodenanordnung, insbesondere im Bereich der zweiten Elektrode, ganz besonders im Bereich von Kanten der zweiten Elektrode, Oberflächen-Mikroentladungen (Surface Micro Discharges - SMD) aus, die wiederum zur Entstehung eines nicht-thermischen Plasmas im Bereich der aktiven Oberfläche führen.

Die erste Elektrode und die zweite Elektrode sind insbesondere als Leistungselektroden ausgebildet.

Unter einem elektrisch leitfähigen Kunststoff werden insbesondere intrinsisch leitfähige Polymere, die auch als leitfähige Polymere bezeichnet werden, verstanden. Diese bilden Kunststoffe, deren elektrische Leitfähigkeit mit der elektrischen Leitfähigkeit von Metallen vergleichbar ist. Zugleich sind solche Kunststoffe sehr leicht. Beispiele für solche elektrisch leitfähigen Kunststoffe sind beispielsweise Poly-3,4-ethylendioxythiofen (PEDOT oder PEDT), insbesondere in Kombination mit Polystyrolsulfonat (PSS) als Gegenion, Polyethin, Polyanilin, Polyparaphenylen (PPP), Polypyrrol (PPy) und dotiertes Polythiophen (PT).

Unter einem Leitkleber wird insbesondere ein elektrisch leitfähiger Klebstoff verstanden. Dieser vereint ebenfalls ein geringes Gewicht mit einer guten elektrischen Leitfähigkeit und einer einfachen Applizierbarkeit.

Das Dielektrikum weist bevorzugt ein Material auf oder besteht aus einem Material, welches ausgewählt ist aus einer Gruppe bestehend aus Kapton, Quarz, Glas, und Keramik, insbesondere Aluminiumoxid.

Das Dielektrikum weist bevorzugt eine - in Stapelrichtung eines aus der ersten Elektrode, dem Dielektrikum und der zweiten Elektrode gebildeten Stapels gemessenene - Dicke von mindestens 0,05 mm bis höchstens 0,8 mm, vorzugsweise von mindestens 0,1 mm bis höchstens 0,75 mm, besonders bevorzugt von 0,25 mm auf.

Die erste Elektrode weist bevorzugt eine - in Stapelrichtung gemessene - Dicke von mindestens 1 $\mu$m bis höchstens 100 $\mu$m, insbesondere eine Dicke von mindestens 2 $\mu$m bis höchstens 6 $\mu$m, insbesondere eine Dicke von 4 $\mu$m auf.

Die zweite Elektrode weist bevorzugt eine - in Stapelrichtung gemessene - Dicke von mindestens 5 $\mu$m bis höchstens 1 mm, bevorzugt eine Dicke von 0,5 mm auf.

Die Elektrodenanordnung kann eine flache Form, aber auch eine gebogene Form aufweisen. Es ist möglich, dass die Elektrodenanordnung starr ausgebildet ist. Die Elektrodenanordnung kann aber bevorzugt auch flexibel, insbesondere biegsam, ausgebildet sein.

Bei einem bevorzugten Ausführungsbeispiel weist die erste Elektrode eine flächenmäßige Ausdehnung von 2 cm bis 5 cm, vorzugsweise 3 cm, mal 2 cm bis 5 cm, vorzugsweise 3 cm, auf und ist bevorzugt quadratisch ausgebildet. Das Dielektrikum und die zweite Elektrode sind bevorzugt ebenfalls quadratisch, und/oder mit einer flächenmäßigen Ausdehnung von 3 cm bis 6 cm, vorzugsweise 4 cm, mal 3 cm bis 6 cm, vorzugsweise 4 cm, ausgebildet. Die erste Elektrode ist vorzugsweise zentral oder mittig relativ zu der Anordnung aus dem Dielektrikum und der zweiten Elektrode angeordnet. Eine spezifische Ausgestaltung der Elektrodenanordnung weist für die erste Elektrode eine flächenmäßige Ausdehnung von 3,4 cm auf 3,4 cm auf.

Ist die erste Elektrode bezüglich ihrer flächigen Erstreckung kleiner als die zweite Elektrode, überragt also die zweite Elektrode die erste Elektrode randseitig, ist die erste Elektrode bevorzugt mit einer Isolationsschicht versehen, die verhindert, dass Mikroentladungen entlang des Rands der ersten Elektrode entstehen.

Gemäß einer Weiterbildung der Erfindung ist vorgesehen, dass die Steuereinrichtung eine mit der Elektrodenanordnung in Reihe schaltbare oder geschaltete elektronische Proxystruktur aufweist, wobei der wenigstens eine Leistungs-Parameter durch die Steuereinrichtung an der elektronischen Proxystruktur erfasst wird. Die elektronische Proxystruktur ist dabei bevorzugt die in Zusammenhang mit dem Verfahren erläuterte elektronische Proxystruktur.

Gemäß einer Weiterbildung der Erfindung ist vorgesehen, dass die elektronische Proxystruktur als Kapazität, insbesondere als Kondensator oder Kondensator-Anordnung, ausgebildet ist.

Gemäß einer Weiterbildung der Erfindung ist vorgesehen, dass die zweite Elektrode eine periodische Struktur aus einer Mehrzahl identischer oder verschiedener Strukturelemente aufweist. Die Strukturelemente bilden einzeln oder in Kombination miteinander Einheitszellen der periodischen Struktur. Die zweite Elektrode ist demnach insbesondere als strukturierte Elektrode ausgebildet. Die Strukturelemente sind miteinander elektrisch verbunden, wobei sie insbesondere auf ein identisches Potential gelegt werden können. Die Strukturelemente können quasi eindimensional ausgebildet sein, beispielsweise als gerade oder auch gekrümmte oder gewundene, linien- oder wellenförmige Teilelektroden, oder sie können zweidimensional ausgebildet sein, beispielsweise als zweidimensionale, durchgängige Struktur wie eine Mäanderstruktur, oder als Polygone, insbesondere als Dreieck, Quadrat, Fünfeck, Sechseck oder höherzähliges Mehreck. Die Strukturelemente können auch als Kreise, Ellipsen, oder Ovale ausgebildet sein. Eine Plasmaentladung bildet sich insbesondere an Kanten der Strukturelemente aus.

Weist die zweite Elektrode eine periodische Struktur aus einer Mehrzahl identischer Strukturelemente auf, ist sie insbesondere skalierbar ausgebildet. Dies bedeutet, dass eine Erzeugungsrate von reaktiven Spezies in einem durch die Elektrodenanordnung erzeugten Plasma bei gleicher spezifischer Leistung, bezogen auf eine Einheitsfläche der Elektrodenanordnung oder auf eine Einheitskantenlänge der Kanten der Strukturelemente der zweiten Elektrode, entlang der Plasma erzeugt wird, linear mit der flächigen oder geometrisch linearen Ausdehnung der zweiten Elektrode und insbesondere mit der Anzahl der identischen Strukturelemente oder der Gesamtkantenlänge der Strukturelemente skaliert. Dies erweist sich als besonders günstig zur Abstimmung der elektrischen, chemischen und/oder mikrobiellen Eigenschaften der Elektrodenanordnung.

Gemäß einer Weiterbildung der Erfindung ist vorgesehen, dass die zweite Elektrode wenigstens ein Strukturelement mit wenigstens einer von Kanten begrenzten Ausnehmung aufweist, wobei die die Ausnehmung begrenzenden Kanten innerhalb jeder Ausnehmung eine Kantenlänge von mindestens 0,5 mm bis höchstens 10 mm, vorzugsweise von mindestens 1 mm bis höchstens 8 mm, vorzugsweise von mindestens 2 mm bis höchstens 7 mm, vorzugsweise von 2 mm oder von 5 mm zueinander aufweisen. Ein solches Strukturelement kann insbesondere als Polygon, beispielsweise als Dreieck, als Viereck, als Fünfeck, als Sechseck, oder als Vieleck mit höherer Anzahl von Ecken ausgebildet sein. Dabei sind die Kanten des Vielecks vorzugsweise von leitfähigem Material der zweiten Elektrode gebildet, wobei innerhalb der von den Kanten begrenzten Ausnehmungen, das heißt insbesondere im Inneren des Mehrecks, kein leitfähiges Material ausgebildet ist. Eine Plasmaentladung bildet sich in diesem Fall entlang der Kanten des Strukturelements aus. Dabei hat sich gezeigt, dass sich die von zwei in einer Ecke des Mehrecks aufeinanderstoßenden Kanten ausgehenden elektrischen Felder im Bereich der Ecke gegenseitig stören, wobei hier insbesondere Selbstinterferenz auftritt. Dies bewirkt eine Verringerung der Leistung der Elektrodenanordnung, insbesondere eine Verringerung der Plasmaerzeugungsrate, weil Verluste entstehen. Diese Verluste sind in Relation zur Plasma-Leistung der Elektrodenanordnung kleiner, wenn die Strukturelemente größer sind, was insbesondere bedeutet, dass die relativen Verluste kleiner werden, je länger die Kantenlänge der Strukturelemente ist. Insbesondere bei den hier angegebenen Werten und ganz bevorzugt ab einer Kantenlänge von 2 mm kann der auf Selbstinterferenz zurückzuführende leistungsmindernde Effekt in Kauf genommen oder vernachlässigt werden.

Die hier beschriebene Selbstinterferenz steht auch einer beliebigen Verfeinerung und damit Steigerung der Flächenleistung der Elektrodenanordnung im Wege. Weist nämlich beispielsweise die zweite Elektrode eine periodische Struktur aus einer Mehrzahl identischer Strukturelemente auf, die jeweils eine von Kanten begrenzte Ausnehmung aufweisen, könnte theoretisch beispielsweise die Erzeugungsrate bei gleicher Gesamtfläche der zweiten Elektrode verdoppelt werden, wenn eine charakteristische Länge der Strukturelemente, beispielsweise die Kantenlänge eines quadratischen Strukturelements, halbiert würde. Hierdurch könnte die Anzahl der Strukturelemente auf der gesamten, konstanten Elektrodenfläche vervierfacht werden, wodurch im Ergebnis die Gesamtlänge der Kanten, also die Summe der Kantenlängen aller Strukturelemente, verdoppelt würde. Hierdurch würde auch die Erzeugungsrate verdoppelt, wenn keine Selbstinterferenz aufträte. Je kleiner jedoch die Strukturelemente werden, das heißt je kürzer ihre Kantenlängen sind, desto stärker leistungsmindernd wirkt sich die Selbstinterferenz in den Ecken aus, sodass die Erzeugungsrate nicht mehr linear mit der Anzahl der Strukturelemente und der Gesamtkantenlänge skaliert. Somit existiert eine effektive Untergrenze für die Kantenlänge der einzelnen Strukturelemente, wobei oben geeignete Bereiche für die Kantenlängen der Strukturelemente angegeben sind.

Es ist auch erkannt worden, dass die Selbstinterferenz in stärkerem Maße auftritt, je kleiner der Winkel zwischen zwei in einer Ecke aufeinandertreffenden Kanten eines Strukturelements ist. Bei einem Dreieck, beispielsweise bei einem gleichschenkligen Dreieck, bei welchem die Kanten unter 60° aufeinandertreffen, wirkt sich die Selbstinterferenz stärker leistungsmindernd aus, als beispielsweise bei einem Quadrat, bei welchem die Kanten unter 90° aufeinandertreffen. Entsprechend sinkt die leistungsmindernde Wirkung der Selbstinterferenz in den Ecken mit der Anzahl der Kanten, welche ein mehreckiges Strukturelement aufweist, oder allgemeiner gesagt mit zunehmendem Winkel, in welchem zwei Kanten eines Strukturelements in einer Ecke aufeinandertreffen.

Die zuvor angegebenen Wertebereiche sind insbesondere für ein quadratisches Strukturelement optimal, können aber ohne weiteres auch für andere, mehreckige Strukturelemente angewendet werden. Allerdings können die Werte grundsätzlich kleiner gewählt werden, je mehr Kanten ein Strukturelement aufweist, oder je größer der Winkel zweier in einer Ecke aufeinandertreffenden Kanten relativ zueinander ist.

Alternativ oder zusätzlich ist bevorzugt vorgesehen, dass die zweite Elektrode eine Mehrzahl von Strukturelementen aufweist, wobei die einzelnen Strukturelemente einen Abstand von mindestens 0,5 mm bis höchstens 10 mm, vorzugsweise von mindestens 1 mm bis höchstens 8 mm, vorzugsweise von 5 mm, zueinander aufweisen. Auch dieser Abstand zwischen den einzelnen Strukturelementen ist relevant, weil insbesondere kleinere Abstände, das heißt feinere Gitter, zu Effizienzverlusten aufgrund von Interferenzeffekten führen können.

Besonders bevorzugt weist die zweite Elektrode ein periodisches Gitter von quadratischen Strukturelementen auf, wobei die Kanten eines quadratischen Strukturelements eine - senkrecht zu ihrer Erstreckung gemessene - Breite von 0,5 mm aufweisen, und wobei eine Aussparung eines solchen quadratischen Strukturelements bevorzugt eine innere Kantenlänge von 5 mm aufweist.

Die Elektrodenanordnung ist gemäß einem bevorzugten Ausführungsbeispiel planar ausgebildet. Es ist aber auch möglich, dass die Elektrodenanordnung halbzylinderförmig ausgebildet ist, wobei bevorzugt die zweite Elektrode Strukturelemente aufweist, deren Größe von einer Mitte, das heißt einem Apex des Halbzylinders, nach außen zunimmt. Hierdurch kann das erzeugte Plasma im mittleren Bereich, das heißt am Apex oder Extremum der halbzylindrischen Elektrodenanordnung konzentriert werden, während in den Randbereichen eine geringere Plasma-Leistung oder Erzeugungsrate vorliegt.

Auch eine kugelförmige Elektrodenanordnung oder halbkugelförmige Elektrodenanordnung ist denkbar. In diesem Fall weist die zweite Elektrode bevorzugt eine fußballartige Struktur auf, die aus einander abwechselnden Pentagonen und Hexagonen gebildet ist.

Die zweite Elektrode kann auch linienförmig, gerade, zick-zack-förmig, bogenförmig, wellig, spiralig, kammartig oder mäanderförmig ausgebildet sein.

Gemäß einer Weiterbildung der Erfindung ist vorgesehen, dass die erste Elektrode Kupfer und/oder Zinn aufweist. Die erste Elektrode besteht alternativ aus Kupfer oder einer Kupferlegierung und/oder aus Zinn oder einer Zinnlegierung. Besonders bevorzugt weist die erste Elektrode eine erste Lage aus Kupfer oder einer Kupferlegierung und eine zweite, auf der ersten Lage angeordnete Lage aus Zinn oder einer Zinnlegierung auf. Dabei ist die Kupferlage bevorzugt dem Dielektrikum zugewandt, wobei die Zinn aufweisende Lage auf der Kupfer aufweisenden Lage angeordnet und dem Dielektrikum abgewandt ist. Die Zinn aufweisende Lage dient dabei insbesondere einer besseren Kontaktierbarkeit der ersten Elektrode. Die Kupfer aufweisende Lage weist demgegenüber eine besonders hohe elektrische Leitfähigkeit und insbesondere eine höhere elektrische Leitfähigkeit als Zinn auf. Die Kupfer aufweisende Lage weist vorzugsweise - in Stapelrichtung gemessen - eine Dicke von 3 $\mu$m auf. Alternativ oder zusätzlich weist die Zinn aufweisende Lage bevorzugt eine - in Stapelrichtung gemessene - Dicke von 1 $\mu$m auf.

Gemäß einer Weiterbildung der Erfindung ist vorgesehen, dass das Dielektrikum und die zweite Elektrode die erste Elektrode allseitig - senkrecht zu der Stapelrichtung gesehen - überragen. Diese Ausgestaltung hat insbesondere mechanische Vorteile beim festen Aufdrücken oder Aufpressen der zweiten Elektrode auf das Dielektrikum. Problematisch ist gegebenenfalls allerdings, dass über die Ränder der ersten Elektrode hinausragende Bereiche der zweiten Elektrode Feldüberhöhungen des elektrischen Feldes erfahren können, wodurch unerwünschte Entladungspfade, beispielsweis durch Kriechströme oder Koronaentladungen, ausgebildet werden. Dies wiederum reduziert die Effizienz der Elektrodenanordnung.

Alternativ ist es möglich, dass die erste Elektrode und das Dielektrikum die zweite Elektrode allseitig - senkrecht zur Stapelrichtung gesehen - überragen. **In** diesem Fall können die zuvor beschriebenen Feldüberhöhungen zumindest weitgehend, insbesondere bis auf einen Kontaktierungsbereich, in welchem die zweite Elektrode kontaktiert ist, vermieden werden, wodurch die Effizient der Elektrodenanordnung hoch ist.

Alternativ ist es auch möglich, dass das Dielektrikum die erste Elektrode und die zweite Elektrode allseitig - senkrecht zur Stapelrichtung gesehen - überragt. **In** diesem Fall ist ein möglicher Entladungspfad von der ersten Elektrode zur zweiten Elektrode oder umgekehrt über die Oberfläche des Dielektrikums besonders lang, sodass Kriechströme und andere, parasitäre Entladungen, beispielsweise Koronaentladungen, wirksam verhindert werden.

Gemäß einer Weiterbildung der Erfindung ist vorgesehen, dass die erste Elektrode mit einer elektrischen Isolierschicht beschichtet und/oder mit einer Vergussmasse vergossen ist. Auf diese Weise kann insbesondere diejenige Elektrode, an welche bestimmungsgemäß eine Spannung angelegt wird, isoliert und insbesondere eingehaust werden, wodurch die elektrische Sicherheit der Elektrodenanordnung erhöht ist. Die Isolierschicht weist bevorzugt einen Isolierlack, insbesondere einen Zwei-komponenten-Isolierlack, auf. Dieser kann auf die erste Elektrode aufgesprüht oder aufgestrichen oder in anderer geeigneter Weise aufgebracht werden. Die Isolierschicht und/oder Vergussmasse dient im Übrigen auch der Vermeidung von Kriechströmen. Insbesondere können sprühfähige Isolierlacke zur Ausbildung der Isolierschicht verwendet werden.

Bevorzugt wird im Rahmen des Verfahrens eine Elektrodenanordnung einer Plasmaquelle geprüft, die zur Erzeugung eines nicht-thermischen Plasmas eingerichtet ist und eine Elektrodenanordnung nach einem der zuvor beschriebenen Beispiele aufweist. Die Plasmaquelle weist außer der Elektrodenanordnung bevorzugt eine Steuereinrichtung zur Ansteuerung der Elektrodenanordnung, insbesondere zur Bestromung derselben, und eine elektronische Speichereinrichtung, nämlich insbesondere die zuvor bereits beschriebene, der Elektrodenanordnung zugeordnete elektronische Speichereinrichtung, auf. Die Steuereinrichtung ist dabei insbesondere eingerichtet, um ein Verfahren gemäß einer der zuvor beschriebenen Ausführungsformen durchzuführen.

Die Plasmaquelle ist bevorzugt als handhaltbares Gerät ausgebildet, welches von einem Verwender vorzugsweise mit einer Hand gehalten und getragen werden kann. Dabei kann die Plasmaquelle insbesondere eine Größe aufweisen, bei der sie einhändig bedient und getragen werden kann, beispielsweise die Größe eines Telefonhörers oder eines Duschkopfs.

Die Plasmaquelle weist weiterhin bevorzugt Mittel auf, um mit einem Verwender zu kommunizieren, wobei diese Mittel vorzugsweise ausgewählt sind aus einer Gruppe bestehend aus akustischen Kommunikationsmitteln, insbesondere einem Lautsprecher, optischen Kommunikationsmitteln, insbesondere Signalleuchten, vorzugsweise Leuchtdioden, einem Anzeigemittel zur Anzeige von Grafiken und/oder Texten, insbesondere wenigstens ein Display, und Vibrationsmitteln zur Erzeugung einer Vibration der Plasmaquelle.

Die Plasmaquelle ist vorzugsweise batterie- oder akkumulatorbetrieben ausgebildet und insoweit kabellos und insbesondere ohne Kontakt zu einer feststehenden, größeren Einrichtung betreibbar. Da das Plasma in Umgebungsluft erzeugt wird, weist die Plasmaquelle bevorzugt auch keine Gasversorgung zur Zufuhr eines Trägergases auf.

Die Plasmaquelle kann aber auch als größeres, stationäres und/oder kabelgebundenes Gerät ausgebildet sein.

Im Folgenden werden grundsätzliche Überlegungen zu dem zuvor beschriebenen Verfahren zum Prüfen einer Elektrodenanordnung zur Erzeugung eines nicht-thermischen Plasmas näher erläutert:

Flächige oder auch lineare Elektrodenanordnungen (z.B. DBD, SMD, beschichtete SMD, unabhängig von ihrer Geometrie) variieren in ihrer Plasma-Leistung in Abhängigkeit von Umweltbedingungen. Das trifft insbesondere für die Luftfeuchtigkeit zu, die einen starken Einfluss abhängig von den verwendeten Materialien, insbesondere von dem Dielektrikum haben kann. Das ist zum Teil begründet durch die chemischen Bedingungen der Luft (z.B. Anregungswahrscheinlichkeiten, Dissoziation, Ionisation), die sich hauptsächlich durch die variierende Luftfeuchtigkeit darstellt, aber auch durch Kondensation auf die Elektroden und Absorption und Diffusion in das dielektrische Material. Letzteres lässt sich durch z.B. "Einbrennen" der Elektrodenanordnung vor jedem Gebrauch reduzieren - ersteres ist ohne Kenntnis der Umweltbedingungen nicht kompensierbar. Hinzu kommt, dass die Elektrodenanordnung durch längeren Gebrauch altern und verschmutzen kann, was die Funktionalität beeinträchtigt. Unter "Alterungseffekte" sind z.B. Korrosion, Ablagerungen, Oberflächenveränderungen zusammengefasst, die bei längerem Gebrauch auftreten können und zu einer Beeinträchtigung der Leistung führen können.

Die Funktionalität der Elektrodenanordnung ist in den meisten Fällen nicht durch eine einfache ja/nein Entscheidung beantwortet, wenn man lediglich feststellt ob Strom fließt. Zum Beispiel für Hygiene, Oberflächenbehandlung, Textilbehandlung, Wasseraufbereitung, Behandlung von Lebensmitteln, Saatgut, Hauterkrankungen und Wunden ist es entscheidend, dass die Plasma-Dosis für den jeweiligen Anwendungszweck ausreicht, um Pathogene (Bakterien, Viren, Sporen) zum gewünschten Grad zu inaktivieren. "Plasma-Dosis" ist das Produkt der Plasmaerzeugungsrate (Plasma-Leistung) multipliziert mit der Zeit. Ebenfalls wichtig ist die Plasmachemie, die steuerbar ist und damit den Anwendungsbereich definiert. Auch die Plasmachemie kann von der Plasma-Leistung abhängig sein (z.B. in der Luft kann entweder Sauerstoff- oder Stickstoffchemie als dominant gewählt werden oder ein Zwischenbereich oder eine sequenzielle variable Behandlung).

Verfahren:

Generell ist die typische Problemstellung wie folgt:
Die ausschlaggebende Eigenschaft der Plasmaquelle ist die Inaktivierung von Pathogenen - vorwiegend Bakterien und/oder die Inaktivierung von Allergenen, Geruchsmolekülen oder sonstigen unerwünschten oder gefährlichen Molekülen (Dekontamination) - bis zu einer geforderten Log-Reduktion in einer vorbestimmten Zeit t.

Beispiel Bakterieninaktivierung in vorgegebener Zeit:

Ad 1. Für die gewünschte Behandlungszeit müssen entsprechende Bakterientests durchgeführt werden. Diese definieren die Plasma-Leistung, die am Gerät voreingestellt wird.

Ad 2. Die dafür ausschlaggebende Messgröße ist die Plasma-Leistung PL - d.h. wie viel Energie wird pro Sekunde in die Erzeugung eines nicht-thermischen kalten atmosphärischen Plasmas gesteckt?

Die Plasmaerzeugung entsteht in der SMD Elektrode in Millionen von Mikroentladungen, die in der Stromkurve als enge "Spikes" zu sehen sind - jeder Spike mit einer typischen Dauer von einigen 10 Nanosekunden. Diese Hochfrequenzkomponente ist z.B. für ein kleines Handgerät nicht direkt messbar, der Aufwand ist zu groß. Für eine größere Anlage ist der Aufwand ebenfalls hoch, aber eher realisierbar - es sei denn ein einfacheres zuverlässiges Verfahren existiert.

Ad 3. Es muss also eine Messgröße gefunden werden, welche durch eine einfache Messung durchführbar ist, die einen direkten Rückschluss auf die Plasma-Leistung PL erlaubt - ein Leistungs-Parameter PM. Dabei muss kein linearer Zusammenhang zwischen dem Leistungs-Parameter PM und der Plasma-Leistung PL bestehen; vielmehr genügt eine eindeutige, vorzugsweise bijektive Beziehung zwischen diesen Größen. Ein linearer Zusammenhang stellt aber eine besonders einfache Ausgestaltung dar, weshalb hier beispielhaft von einem solchen ausgegangen wird.

Dieser Leistungs-Parameter PM sollte für alle Umweltbedingungen gelten - d.h. PM/PL sollte idealerweise konstant sein, unabhängig von Temperatur und Luftfeuchtigkeit. Dann kann PM benutzt werden, um die Plasma-Leistung PL der Plasmaquelle eindeutig in jeder Anwendung zu identifizieren - durch einen einfachen ersten "Skalierungsfaktor" S1 = PL/PM.

Ad 4. Die Elektrodenanordnung kann "Alterungseffekte" aufweisen, die nicht die Effektivität beeinflussen, wohl aber die Plasma-Leistung verändern können.

Das Verhältnis S1 = PL/PM sollte auch für Elektrodenanordnungen mit Alterungserscheinungen idealerweise konstant sein, wobei dieses Verhältnis im optimalen Fall immer gleich sein sollte, unabhängig vom Alter der Elektrodenanordnung. Das ist allerdings nicht zu erwarten, weil die Elektrodenanordnungen sich (z. B. durch Korrosion, Oxidation, Ablagerung) verändern können. Daher müssen Alterungseffekte in Langzeittests untersucht werden, und ggf. muss die Elektrodenanordnung nach einer bestimmten Zahl von Anwendungen ausgetauscht werden.

Ad 5. Falls die Messung unter anderen Umweltbedingungen stattfindet als die endgültigen Betriebsbedingungen (für einen Funktionstest vor der Anwendung nicht unüblich) muss die Skalierung von den Umwelt- zu den Betriebsbedingungen ebenfalls experimentell bestimmt werden.

Das ist zum Beispiel der Fall, wenn ein Funktionstest im normalen Raum-Umfeld durchgeführt wird, die Anwendung aber in einem feuchten geschlossenen Volumen stattfindet (das entspricht z.B. der Wasseraufbereitung oder ggf. der Wundbehandlung). Dazu wird ein zusätzlicher zweiter Skalierungsfaktor (S2) benötigt, der experimentell bestimmt wird. Die Skalierung von der "Proxy Messung" des Leistungs-Parameters PM zum endgültigen Anwendungsbereich ist dann als Gesamt-Skalierungsfaktor S aus dem Produkt S1xS2 = S gegeben.

Ad 6. Die Funktionalitätsprüfung der Elektrodenanordnung kann generell auch unter Umweltbedingungen (Temperatur, Relative Luftfeuchtigkeit) erfolgen, die nicht bekannt sind.

Damit die Skalierung zum Anwendungsbereich trotzdem benutzt werden kann ist es wichtig, dass das Verhältnis PL/PM keine große Variabilität mit Luftfeuchtigkeit aufweist. Zusätzlich empfiehlt es sich einen "Standardbereich" für Luftfeuchtigkeit zu bestimmen (in welchem mit hoher Wahrscheinlichkeit die Funktionalität geprüft wird) und von diesem ausgehend die Skalierung durchzuführen.

Ad 7. Vor der Anwendung wird die Elektrodenanordnung zunächst ggf. "eingebrannt", um Lagerungseffekte (z.B. durch Kondensation) zu entfernen. Dann wird die Messung des Leistungs-Parameters PM durchgeführt - und zwar unter den jeweils vorherrschenden (nur näherungsweise bekannten oder sogar unbekannten) Umweltbedingungen.

Der Skalierungsfaktor S wird auf diese Messung angewendet, um die berechnete Proxy-Plasma-Leistung unter Betriebsbedingungen zu erhalten. Die intra-individuelle und inter-individuelle Variation in den Elektrodenanordnungen muss als "Bandbreite" des Skalierungsfaktors Berücksichtigung finden.

Ad 8. Eine weitere Komplikation - die Elektrode kann teil-verschmutzt sein, so dass die Plasma-Leistung gegenüber einer sauberen Elektrode reduziert ist.

Es muss sichergestellt sein, dass die Werte PM und PL in gleicher Weise verknüpft sind - d.h. S1 ist idealerweise unabhängig vom Verschmutzungsgrad. Das gleiche trifft auf den zweiten Skalierungsfaktor S2 zu. Auch diese Relationen müssen experimentell verifiziert werden.

Ad 9. Im vorliegenden Fall (insbesondere medizinische Anwendung oder Wasseraufbereitung) gibt es noch eine weitere Komplikation. Die eigentliche "Messgröße" ist nicht die Plasma-Leistung,- es ist die bakterizide Wirkung des Plasmas, wie schon zu Anfang angemerkt. Diese wird durch die integrierte Plasma-Dosis bei einem voreingestellten Betriebsmodus beschrieben. Die Anwendungsdauer, welche die erforderliche Minimaldosis mit einem zusätzlichen Sicherheitsfaktor übertrifft, ist durch entsprechende Tests vorbestimmt und soll immer gleichbleiben. Damit wird die "Plasma-Dosis" nur durch die Plasma-Leistung im Anwendungsbereich definiert, die über die o.g. Schritte bestimmt werden kann.

Um eine erfolgreiche Behandlung zu garantieren, muss ein unterer Schwellenwert, PSU, für die Plasma-Leistung im Anwendungsfall experimentell bestimmt werden. Der im Funktionalitätstest erfasste Schwellenwert für PM ist dann PU = PSU/S. Liegt die Plasma-Leistung PL unter dem Schwellenwert PSU, funktioniert die Elektrodenanordnung nicht gut genug, um den geforderten Effekt (z.B. 3-4 Log bakterizide Effektivität) zu erreichen.

Sofern der Gesamt-Skalierungsfaktor S und der Schwellenwert PSU hinreichend sorgfältig gewählt wurden, so dass alle Fertigungstoleranzen und erwarteten Umweltveränderungen gegenüber den (unbekannten) Umweltbedingungen beim Funktionstest einbezogen sind, gibt der so bestimmte Wert PM > PU eine Garantie, dass die Plasma-Leistung ausreicht, um den geforderten Zweck zu erfüllen.

Die erste Bedingung für eine positive Funktionalität ist also:

PM > PU, wobei der untere Schwellenwert PU = PSU/S.

Eine zweite Bedingung für eine positive Funktionalität ist durch einen oberen Schwellenwert, PSO, gegeben, der nicht überschritten werden darf. Durch verschiedene Störungen (z.B. altersbedingte Erosion des Dielektrikums, Deposition von leitenden Erosionsprodukten auf dem Dielektrikum, Bildung von Kriechströmen usw.) kann die Plasma-Leistung über den normalen Betriebswert steigen. Dann ist die Elektrodenanordnung zwar im Prinzip noch einsatzfähig, aber es sind zwei wichtige Änderungen, die zu berücksichtigen sind:

A. Die Elektrodenanordnung ist geschädigt und/oder modifiziert und damit nicht mehr vergleichbar mit der Elektrodenanordnung die als Referenzmessung (z.B. in Vorversuchen, Labor oder in der Präklinik) verwendet wurde;

B. Die Elektrodenanordnung könnte vom Sauerstoff-Modus in den Stickstoff Modus (bei genügend stark erhöhter Leistung) eintreten. Das ändert die bakterizide Wirkung ebenfalls und ist mit den Vorversuchen, Labormessungen oder Präklinik-Tests nicht kompatibel.

Die zweite Bedingung für eine positive Funktionalität ist also:

PM < PO, wobei der obere Schwellenwert PO = PSO/S.

Um diese logische Kette zu erfassen, und daraus für alle Umweltbedingungen sichere Betriebsparameter Schwellenwerte "PSU und PSM" zu generieren, sind sehr viele Messungen nötig.

Die Plasma-Leistung PL muss unabhängig mit dem entsprechenden Aufwand gemessen werden, und sowohl PL als auch PM müssen für alle relevanten Umweltbedingungen bestimmt und in Relation gesetzt werden. Das muss für unterschiedliche Verschmutzungsgrade und Alterungszustände der Elektrodenanordnung wiederholt werden. Alle so generierten Parameter müssen anhand von Bakterienuntersuchungen mit ihrer bakteriziden Wirkung BE korreliert werden. Wenn all diese Datensätze vorliegen, kann der akzeptable Schwellenwert PS bestimmt werden.

Damit intra-individuelle Schwankungen (z.B. durch Fertigungstoleranzen) ebenfalls einbezogen werden, müssen diese Messungen für eine repräsentative Menge von Elektrodenanordnungen ausgeführt und die Schwankungen (zu typisch 3 σ) berücksichtigt werden.

Beispiel: Die erforderliche Anzahl von Messungen (PM und PL bei 3 Temperaturwerten mit jeweils 7 Luftfeuchtigkeitswerten, dazu jeweils 3 Bakterienmessungen und Kontrollen) für 4 Verschmutzungsgrade - ergibt ca 700 Messwerte pro Gerät - also für die erforderliche Sicherheitsstatistik mehr als 7.000 Messungen!

Aus einer Serie solcher Messungen wird der Schwellenwert für die Plasma-Leistung bestimmt, die erforderlich ist um in der vorgegebenen Behandlungsdauer von 1 Minute den gewünschten bakteriziden Effekt zu erzeugen.

Für eine bevorzugte Elektrodenanordnung wurde der Schwellenwert mit 1 Watt bestimmt.

Der Verschmutzungsgrad kann dabei wichtig sein. Ein bestimmtes Ausführungsbeispiel der Elektrodenanordnung funktioniert noch bei einer Verschmutzung von mehr als 50%. Erst bei 80% Verschmutzung ist die bakterizide Wirkung fast nicht mehr feststellbar.

Es sollte noch erwähnt werden, dass jede neue Elektrodenanordnung mit ähnlichem Aufwand getestet werden muss, um einen zuverlässigen Funktionstest, der für alle relevanten Umweltbedingungen zutrifft, zu ermöglichen.

Natürlich muss jede neue Plasmaquelle (Elektrodenanordnung, Hochspannungsquelle und Steuereinrichtung) auf Sicherheitsaspekte entsprechend den Vorgaben prä-klinisch und in klinischen Studien getestet werden.

Die Erfindung wird im Folgenden anhand der Zeichnung näher erläutert. Dabei zeigen:

Figur 1    eine schematische Darstellung einer Ausführungsform eines Verfahrens zum Prüfen einer Elektrodenanordnung in Form eines Flussdiagramms;

Figur 2    eine schematische Darstellung eines Ausführungsbeispiels einer Plasmaquelle;

Figur 3    eine schematische Querschnittsdarstellung eines Ausführungsbeispiels einer Elektrodenanordnung einer solchen Plasmaquelle, und

Figur 4    eine schematische Draufsicht auf eine solche Elektrodenanordnung.

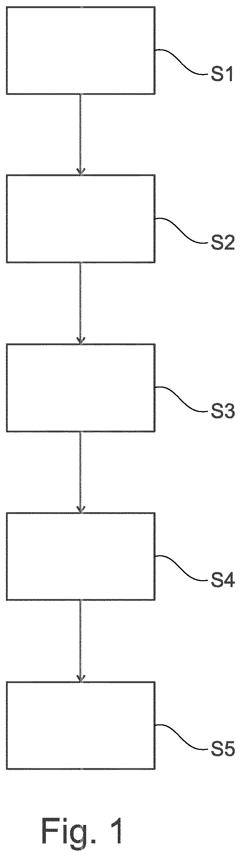

**Fig. 1** zeigt eine schematische Darstellung einer Ausführungsform eines Verfahrens zum Prüfen einer Elektrodenanordnung zur Erzeugung eines nicht-thermischen Plasmas in Form eines Flussdiagramms. Dabei wird in einem ersten Schritt S1 die Elektrodenanordnung in Betrieb genommen, wobei insbesondere eine die Elektrodenanordnung aufweisende Plasmaquelle eingeschaltet wird.

In einem zweiten Schritt S2 wird wenigstens ein Leistungs-Parameter bestimmt, der eine Plasma-Leistung der Elektrodenanordnung charakterisiert.

In einem dritten Schritt S3 wird der wenigstens eine Leistungs-Parameter mit wenigstens einem vorbestimmten Soll-Parameterwert verglichen, woraus ein Vergleichsergebnis erhalten wird.

In einem vierten Schritt S4 wird eine Funktionsfähigkeit der Elektrodenanordnung anhand des Vergleichsergebnisses beurteilt.

Bevorzugt wird in einem fünften Schritt S5 eine Aktion in Abhängigkeit von dem Vergleichsergebnis ausgewählt. Diese Aktion umfasst - abhängig von dem Vergleichsergebnis - bevorzugt die Ausgabe eines "In-Ordnung"-Signals, die Ausgabe eines "Handlungsbedarf"-Signals, die Ausgabe eines "Nicht-In-Ordnung"-Signals, die Mitteilung einer momentanen Plasma-Leistung an einen Betreiber der Elektrodenanordnung, die Anpassung einer Betriebsdauer der Elektrodenanordnung an das Vergleichsergebnis, das Beenden des Betriebs der Elektrodenanordnung, oder ein Fortsetzen des Betriebs der Elektrodenanordnung ohne weitere Maßnahme, insbesondere ohne Signal- oder Mitteilungsausgabe. Die Signalausgabe kann insbesondere in Form von Licht- oder Leuchtsignalen, beispielsweise dem Aktivieren einer grünen, gelben oder roten Leuchte, insbesondere einer LED, erfolgen. Alternativ oder zusätzlich kann ein Text oder ein grafisches Symbol in einem Display angezeigt werden. Auch eine akustische Ausgabe einer Mitteilung oder Warnung ist möglich, sowie ebenfalls die Ausgabe einer Mitteilung oder Warnung durch Erzeugung einer gezielten Vibration der Elektrodenanordnung, insbesondere der Plasmaquelle, welche die Elektrodenanordnung aufweist. Zur Auswahl der Aktion sind bevorzugt vorbestimmte Bereiche für die Übereinstimmung oder Abweichung des wenigstens einen Leistungs-Parameters mit/von dem wenigstens einen vorbestimmten Soll-Parameterwert definiert, wobei die Aktion abhängig davon ausgewählt wird, in welchen der vorbestimmten Bereiche das Vergleichsergebnis fällt.

Bevorzugt wird das Verfahren unmittelbar nach einer Inbetriebnahme, besonders bevorzugt nach jeder Inbetriebnahme der Elektrodenanordnung durchgeführt.

Der wenigstens eine vorbestimmte Soll-Parameterwert kann in einer nicht zur Erfindung gehörenden Ausgestaltung konstant vorgegeben werden. Erfindungsgemäß wird der wenigstens eine vorbestimmte Soll-Parameterwert in Abhängigkeit von wenigstens einem Einsatzparameter der Elektrodenanordnung ausgewählt, wobei er in einem Kennfeld hinterlegt ist. Der wenigstens eine Einsatz-Parameter umfasst eine Umgebungstemperatur der Elektrodenanordnung und/oder eine relative Luftfeuchte in einer Umgebung, insbesondere einer unmittelbaren Umgebung, ganz besonders einer Behandlungsumgebung der Elektrodenanordnung, das heißt einer Umgebung, in welcher mittels dem von der Elektrodenanordnung erzeugten nicht-thermischen Plasma eine Behandlung, insbesondere eine Oberflächenbehandlung, durchgeführt wird. In Abhängigkeit der relativen Luftfeuchte können insbesondere zwei verschiedene Werte für den wenigstens einen vorbestimmten Soll-Parameterwert hinterlegt sein, insbesondere ein erster Wert für eine Luftfeuchte von weniger als 80 %, und ein zweiter, von dem ersten Wert verschiedener Wert für eine relative Luftfeuchte von mehr als 80 %.

Das Plasma wird durch die Elektrodenanordnung insbesondere in Umgebungsluft erzeugt, sodass die relative Luftfeuchte in der Umgebung der Elektrodenanordnung relevant für die Plasmaerzeugung ist.

Die Elektrodenanordnung wird bevorzugt für die Ermittlung des Leistungs-Parameters zumindest bereichsweise beheizt, wobei sie insbesondere auf eine Temperatur von mindestens 50 °C erwärmt werden kann. Auf diese Weise kann auf der Oberfläche der Elektrodenanordnung angelagerte Feuchtigkeit entfernt werden, die ansonsten gegebenenfalls die Messung beeinträchtigen könnte.

Bevorzugt wird das Vergleichsergebnis und/oder der wenigstens eine Leistungs-Parameter für einen späteren Abruf in einer elektronischen Speichereinrichtung protokolliert. Dabei wird/werden das Vergleichsergebnis und/oder der wenigstens eine Leistungs-Parameter bevorzugt mit wenigstens einem Metadatum, insbesondere zusammen mit einem Einsatzort, einem Einsatzzweck, einem Zeitstempel und/oder weiteren Metadaten, vorzugsweise automatisiert, gespeichert. Diese Parameter können dann zu einem späteren Zeitpunkt ausgelesen und/oder grafisch dargestellt werden, um den Betrieb der Elektrodenanordnung zu überwachen und deren Funktionsfähigkeit über die Zeit zu beurteilen.

Die Elektrodenanordnung ist bevorzugt eingerichtet zur Erzeugung von Oberflächenmikroentladungen in Umgebungsluft.

Bevorzugt wird eine Elektrodenanordnung verwendet, die eine erste Elektrode und eine zweite Elektrode aufweist, wobei die erste Elektrode und die zweite Elektrode durch ein Dielektrikum voneinander beabstandet und insbesondere auf verschiedenen Seiten des Dielektrikums in mechanischem Kontakt zu dem Dielektrikum vorgesehen sind. Die erste Elektrode und die zweite Elektrode sind dabei bevorzugt flächig ausgebildet. Die zweite Elektrode ist bevorzugt als Strukturelektrode oder strukturierte Elektrode ausgebildet, die eine Mehrzahl von Kanten aufweist, an denen Oberflächenmikroentladungen gezündet werden können.

Bevorzugt wird an die erste Elektrode eine Hochspannung angelegt, insbesondere eine Wechselspannung, wobei die zweite Elektrode geerdet oder auf Masse gelegt wird. Bei einer Verwendung der Elektrodenanordnung zur Behandlung einer Oberfläche ist bevorzugt die zweite Elektrode der zu behandelnden Oberfläche zugewandt, was die elektrische Sicherheit des Betriebs der Elektrodenanordnung erhöht.

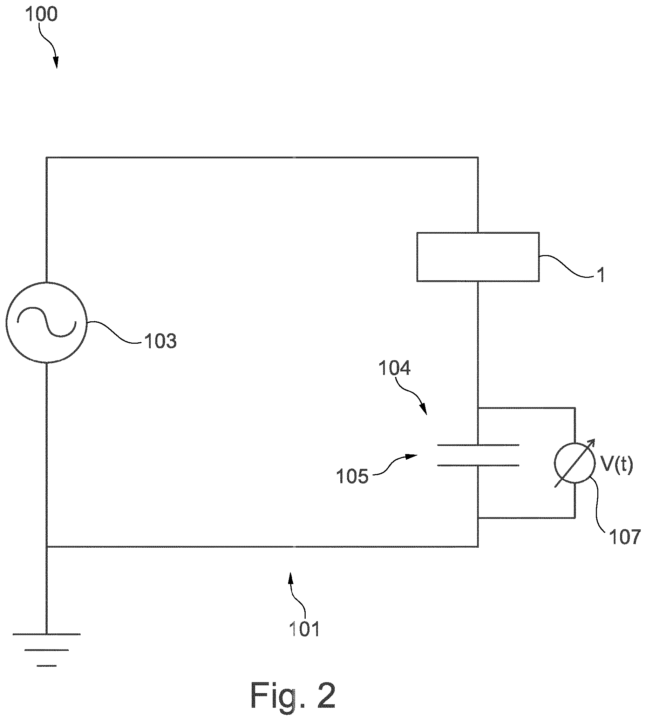

**Fig. 2** zeigt eine schematische Darstellung eines Ausführungsbeispiels einer Plasmaquelle 100 mit einer nur schematisch dargestellten Elektrodenanordnung 1 zum Erzeugen eines nicht-thermischen Plasmas. Die Plasmaquelle 100 weist außerdem eine Steuereinrichtung 101 auf, die eingerichtet ist zur Ansteuerung der Elektrodenanordnung 1. Die Steuereinrichtung 101 weist hier insbesondere eine Spannungsquelle 103 auf, mittels derer eine Wechselspannung als Ansteuerspannung an die Elektrodenanordnung 1 anlegbar ist.

Außerdem weist die Steuereinrichtung eine elektronische Proxystruktur 104 auf, die in Reihe mit der Elektrodenanordnung 1 schaltbar, hier geschaltet ist. Die Steuereinrichtung 101 ist eingerichtet, um den wenigstens einen Leistungs-Parameter an der zu der Elektrodenanordnung 1 in Reihe geschalteten elektronischen Proxystruktur 104 zu erfassen. Die elektronische Proxystruktur 104 ist hier insbesondere als Kapazität 105 ausgebildet.

Als Leistungs-Parameter wird wenigstens ein Wert, insbesondere ein Mittelwert, einer über der elektronischen Proxystruktur 104 zu einem bestimmten Phasenwinkel der Ansteuerspannung abfallenden Wechselspannung V(t) - der Proxyspannung - gemessen, insbesondere über eine Mehrzahl von Perioden der Ansteuerspannung gemittelt, insbesondere gemäß der oben angegebenen Gleichung (4). Dabei wird bevorzugt die Proxyspannung durch eine Spannungsmesseinrichtung 107 zeitabhängig erfasst.

Der Leistungs-Parameter wird vorzugsweise mit einem ersten, oberen Soll-Parameterwert und einem zweiten, unteren Soll-Parameterwert verglichen, wobei die wenigstens eine Aktion davon abhängig gewählt wird, ob der wenigstens eine Leistungs-Parameter in einen durch den ersten Soll-Parameterwert und den zweiten Soll-Parameterwert begrenzten Soll-Parameterbereich fällt.

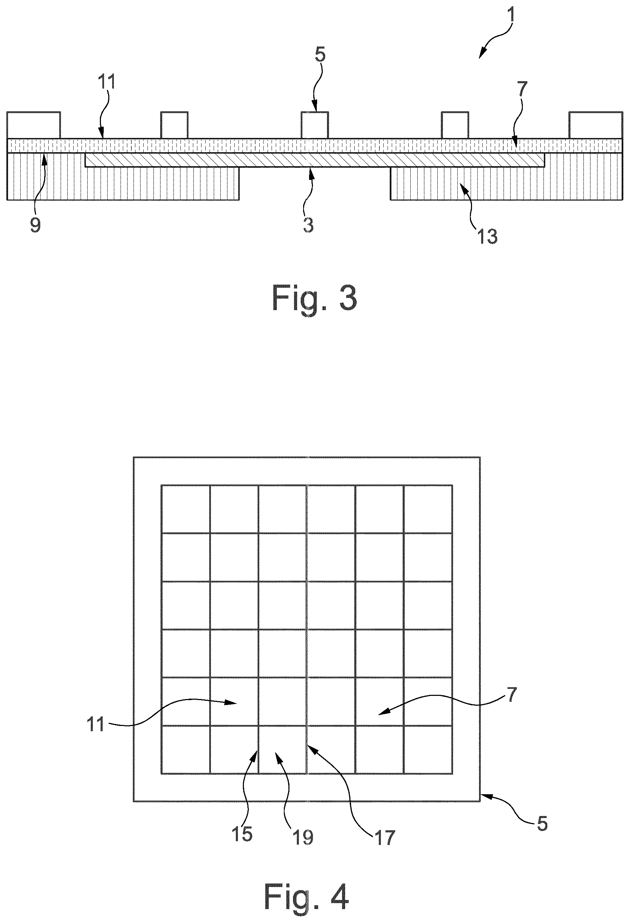

**Fig. 3** zeigt eine schematische Querschnitts- und Ausschnittsdarstellung eines Ausführungsbeispiels einer Elektrodenanordnung 1, die eingerichtet ist zum Erzeugen eines nicht-thermischen Plasmas. Die Elektrodenanordnung 1 weist eine erste Elektrode 3 und eine zweite Elektrode 5 auf, sowie ein Dielektrikum 7, durch welches die erste Elektrode 3 und die zweite Elektrode 5 voneinander beabstandet sind. Insbesondere ist das Dielektrikum 7 - entlang einer Stapelrichtung gesehen - zwischen der ersten Elektrode 3 und der zweiten Elektrode 5 angeordnet. Die Stapelrichtung erstreckt sich in Figur 3 in vertikaler Richtung.

Die erste Elektrode 3 ist hier dicht an einer ersten Seite 9 des Dielektrikums 7 angeordnet, und die zweite Elektrode 5 ist dicht an einer zweiten, der ersten Seite 9 gegenüberliegenden Seite 11 des Dielektrikums 7 angeordnet.

Die zweite Elektrode 5 weist ein Material auf, das ausgewählt ist aus einer Gruppe, bestehend aus Edelstahl, Titan, Wolfram, einem elektrisch leitfähigen Kunststoff, und einem Leitkleber. Außerdem ist die zweite Elektrode 5 gegen die zweite Seite des Dielektrikums 7 gedrängt, insbesondere gegen die zweite Seite 11 angedrückt, auf die zweite Seite 11 aufgepresst, oder allgemein unter Vorspannung an der zweiten Seite 11 des Dielektrikums 7 gehalten.

Die Elektrodenanordnung 1 kann auf einfache, kostengünstige Weise hergestellt werden und weist eine hohe Effizienz sowie eine hohe Beständigkeit, insbesondere gegenüber Oxidation durch Ozon, sowie gegen Sputtering, auf.

Die erste Elektrode 3 weist vorzugsweise Kupfer und/oder Zinn auf. Es ist auch möglich, dass die erste Elektrode 3 aus Kupfer oder einer Kupferlegierung, und/oder aus Zinn oder einer Zinnlegierung besteht. Besonders bevorzugt weist die erste Elektrode 3 eine erste Lage aus Kupfer oder einer Kupferlegierung und eine zweite, auf der ersten Lage angeordnete Lage aus Zinn oder einer Zinnlegierung auf. Dabei ist die zweite Lage aus Zinn oder einer Zinnlegierung insbesondere auf einer dem Dielektrikum 7 abgewandten Seite der ersten Elektrode 3 angeordnet, hier in Figur 3 also an einer Unterseite der ersten Elektrode 3.

Eine in Stapelrichtung gemessene Dicke der ersten Elektrode 3 beträgt vorzugsweise von mindestens 1 $\mu$m bis höchstens 100 $\mu$m, besonders bevorzugt 4 $\mu$m, wobei bevorzugt die Kupferlage der ersten Elektrode 3 eine Dicke von 3 $\mu$m aufweist, wobei die Zinn-Lage der ersten Elektrode 3 eine Dicke von 1 $\mu$m aufweist.

Das Dielektrikum 7 weist vorzugsweise ein Material auf oder besteht aus einem Material, das ausgewählt ist aus einer Gruppe bestehend aus Kapton, Quarz, Glas, Keramik, und Aluminiumoxid. Es weist bevorzugt eine in Stapelrichtung gemessene Dicke von mindestens 0,05 mm bis höchstens 0,8 mm, vorzugsweise von mindestens 0,1 mm bis höchstens 0,75 mm, vorzugsweise von 0,25 mm auf.

Die zweite Elektrode 5 weist vorzugsweise eine in Stapelrichtung gemessene Dicke von mindestens 5 $\mu$m bis höchstens 1 mm, bevorzugt von 0,5 mm auf.

Die zweite Elektrode 5 und das Dielektrikum 7 weisen vorzugsweise eine flächenmäßige Ausdehnung von 4x4 cm$^2$ auf. Die bevorzugt zentral, das heißt insbesondere mittig, an dem Dielektrikum 7 angeordnete erste Elektrode 3 weist vorzugsweise eine flächenmäßige Ausdehnung von 3x3 cm$^2$ auf. Auch andere Größen sind für die Elektrodenanordnung möglich, da diese insbesondere modular und ganz besonders bevorzugt skalierbar ausgebildet ist.

Die hier dargestellte Elektrodenanordnung 1 ist insbesondere flach ausgebildet und bevorzugt eben. Es ist aber auch möglich, dass die Elektrodenanordnung gebogen ausgebildet ist. Die Elektrodenanordnung 1 kann starr und/oder flexibel ausgebildet sein.

Die erste Elektrode 3 ist bevorzugt zumindest bereichsweise mit einer elektrischen Isolierschicht 13 beschichtet. Die Isolierschicht 13 weist vorzugsweise einen Isolierlack auf oder besteht aus einem Isolierlack. Besonders bevorzugt ist sie auf die erste Elektrode 3 aufgesprüht. Insbesondere kann die Isolierschicht 13 aus einem Zweikomponenten-Isolierlack gebildet sein. Sie weist vorzugsweise eine Dicke von mehr als 3 $\mu$m auf. Alternativ oder zusätzlich ist es auch möglich, dass die erste Elektrode 13 mit einer Vergussmasse vergossen ist.

Die erste Elektrode 3 ist vorzugsweise auf das Dielektrikum 7 beschichtet, insbesondere aufgedampft. Insoweit unterscheidet sie sich bevorzugt von der zweiten Elektrode 5, die unter Vorspannung an dem Dielektrikum 7 gehalten und insbesondere gegen die zweite Seite 11 gedrängt ist.

Bei dem hier dargestellten Ausführungsbeispiel überragen das Dielektrikum 7 und die zweite Elektrode 5 die erste Elektrode 3 bevorzugt allseitig - senkrecht zu der Stapelrichtung gesehen. Es ist alternativ auch möglich, dass die erste Elektrode 3 und das Dielektrikum 7 die zweite Elektrode 5 allseitig senkrecht zur Stapelrichtung überragen. Weiter ist es alternativ auch möglich, dass das Dielektrikum 7 sowohl die erste Elektrode 3 als auch die zweite Elektrode 5 senkrecht zur Stapelrichtung allseitig überragt.

**Fig. 4** zeigt eine Draufsicht auf die Elektrodenanordnung 1, insbesondere auf das Ausführungsbeispiel der Elektrodenanordnung 1 gemäß Figur 3. Gleiche und funktionsgleiche Elemente sind mit gleichen Bezugszeichen versehen, sodass insofern auf die vorangegangene Beschreibung verwiesen wird. Der Blick des Betrachters ist dabei auf die zweite Elektrode 5 sowie die zweite Seite 11 des Dielektrikums 7 gerichtet. Die erste Elektrode 3 und die Isolierschicht 13 sind dem Blick des Betrachters verborgen, da sie unterhalb der zweiten Elektrode 5 und dem Dielektrikum 7 angeordnet sind.

Die zweite Elektrode 5 weist bevorzugt eine periodische Struktur aus einer Mehrzahl identischer Strukturelemente 15 auf, von denen hier nur eines mit einem Bezugszeichen versehen ist, um die Übersichtlichkeit zu erhöhen. Die Strukturelemente 15 sind hier als Quadrate ausgebildet. Solche Strukturelemente 15 können aber auch allgemein als Polygone, Dreiecke, Vierecke, Pentagone, Hexagone, oder höherrangige Polygone, als Kreise oder Ellipsen, oder auch als eindimensionale Formen, beispielsweise als Linien, insbesondere als gerade Linien, Wellenlinien, anderweitig gekrümmte Linien oder dergleichen, ausgebildet sein. Auch Formen im Übergangsbereich zwischen einer eindimensionalen und einer zweidimensionalen Ausgestaltung, beispielsweise mäanderartige Strukturen, können für die Strukturelemente 15 gewählt werden. Eine periodische Ausgestaltung der zweiten Elektrode 5 ermöglicht in besonderer Weise eine Skalierbarkeit der Elektrodenanordnung 1, wobei sich deren Erzeugungsrate für das nicht-thermische Plasma quasi linear mit der Anzahl der Strukturelemente 15 skalieren lässt.

Unabhängig davon, ob die zweite Elektrode 5 eine periodische Struktur aus einer Mehrzahl identischer Strukturelemente 15 aufweist, oder ob lediglich ein Strukturelement 15 oder eine Mehrzahl voneinander - insbesondere in Hinblick auf eine Größe und/oder Form - verschieden ausgestalteter Strukturelemente 15 vorgesehen sind, weist die zweite Elektrode 5 bevorzugt wenigstens ein Strukturelement 15 mit wenigstens einer von Kanten 17 begrenzten Ausnehmung 19 auf, wobei hier der besseren Übersichtlichkeit wegen jeweils nur eine Kante 17 und eine Ausnehmung 19 mit einem Bezugszeichen versehen sind. Die die Ausnehmungen 19 begrenzenden Kanten 17 weisen - innerhalb einer Ausnehmung 19 gemessen - bevorzugt eine Kantenlänge von mindestens 0,5 mm bis höchstens 10 mm, vorzugsweise von mindestens 1 mm bis höchstens 8 mm, vorzugsweise von mindestens 2 mm bis höchstens 7 mm, vorzugsweise von 5 mm, auf. Insbesondere weisen die hier quadratischen Ausnehmungen 19 bevorzugt eine flächenmäßige Ausnehmung von 5x5 mm$^2$ auf. Die hier beschriebene Ausgestaltung verringert in vorteilhafter Weise den Einfluss von Selbstinterferenzen des elektrischen Feldes in Ecken der Ausnehmungen 19, welche ansonsten den Wirkungsgrad der Elektrodenanordnung 1 in relevanter Weise reduzieren würden.

Eine Stegbreite der Kanten 17 - senkrecht zur Stapelrichtung und senkrecht zur Längserstreckung einer Kante gemessen - beträgt bevorzugt 0,5 mm. Bei einer anderen bevorzugten Ausgestaltung der Elektrodenanordnung 1 ist bevorzugt vorgesehen, dass die zweite Elektrode 5 eine Mehrzahl von Strukturelementen 15 aufweist, wobei die einzelnen Strukturelemente 15 einen Abstand von mindestens 0,5 mm bis höchstens 10 mm, vorzugsweise von mindestens 1 mm bis höchstens 8 mm, vorzugsweise von 5 mm, zueinander aufweisen. Auch dies hilft dabei, den Effekt von Selbstinterferenzen zu reduzieren.

Die Elektrodenanordnung 1 wird bevorzugt betrieben, indem an die erste Elektrode 3 eine Wechselspannung mit einer Amplitude von mindestens 2 kV$_{pp}$ bis höchstens 5 kV$_{pp}$ und einer Frequenz von mindestens 2 kHz bis höchstens 60 kHz, vorzugsweise von 4 kHz, angelegt wird. Die zweite Elektrode 5 wird bevorzugt auf Masse gelegt.

Im Folgenden werden beispielhaft Werte für die Leistungsdichte der Elektrodenanordnung 1 in den verschiedenen Betriebszuständen genannt, die sich auf ein durch den Abstandshalter umschlossenes Volumen von ungefähr 12,5 cm$^3$ beziehen. Bei anderen eingeschlossenen Volumina sind diese Werte anders zu wählen, um gleiche Betriebszustände zu erhalten: Die Elektrodenanordnung 1 wird vorzugsweise in einem ersten Betriebszustand mit einer Leistung von weniger als 0,01 W/cm betrieben. In diesem ersten Betriebszustand dominieren Sauerstoff-Spezies die Zusammensetzung des nicht-thermischen Plasmas, welches durch die Elektrodenanordnung 1 in Umgebungsluft erzeugt wird. In einem dritten Betriebszustand wird die Elektrodenanordnung 1 bevorzugt mit einer Leistung von mehr als 0,05 W/cm betrieben. In diesem dritten Betriebszustand dominieren Stickstoff-Spezies die Zusammensetzung des nicht-thermischen Plasmas. In einem zweiten, intermediären Zustand wird die Elektrodenanordnung 1 bevorzugt mit einer Leistung von mindestens 0,01 W/cm bis höchstens 0,05 W/cm betrieben. In diesem intermediären Zwischenzustand sind sowohl aktive Sauerstoff-Spezies als auch aktive Stickstoff-Spezies in relevanter Konzentration von dem nicht-thermischen Plasma umfasst, wobei das Verhältnis zwischen Stickstoff-Spezies und Sauerstoff-Spezies durch Variation der Leistungsaufnahme der Elektrodenanordnung 1 verändert werden kann.

Die Elektrodenanordnung 1 wird bevorzugt für eine erste vorbestimmte Zeit in dem ersten Betriebszustand, und nach Ablauf der vorbestimmten Zeit für eine zweite, vorbestimmte Zeit in dem zweiten Betriebszustand oder in dem dritten Betriebszustand betrieben.

Die Elektrodenanordnung 1 wird bevorzugt verwendet zur Inaktivierung von pathogenen Keimen, insbesondere von Bakterien, Pilzinfektionen, insbesondere Haut- und/oder Fußpilz, Prionen, Biofilmen und/oder Viren. Diese können insbesondere auf Oberflächen, seien es unbelebte Oberflächen oder Oberflächen von Lebewesen, insbesondere von Pflanzen, Tieren und/oder Menschen, inaktiviert werden. Dies trifft besonders zu auf Hautoberflächen zum Zwecke der Desinfektion oder Sterilisation, und/oder zur Wundbehandlung.

Eine große Serie von Messungen wurde in einer Umweltkammer durchgeführt, um die Korrelation zwischen der "echten Plasma-Leistung" und der "Proxy-Messung" mit dem in Figur 2 gezeigten Schaltbild zu bestimmen.

Das Ergebnis von hunderten solcher Messungen zeigt, dass es eine sehr gute Korrelation zwischen der echten und der Proxy-Bestimmung der Plasma-Leistung gibt und dass die Variation zwischen verschiedenen Plasmaquellen 100 und/oder Elektrodenanordnungen 1 gleicher Bauart sehr gering ist.

Die gute Korrelation besteht bei allen Umweltbedingungen, die im Testbereich lagen.

Mit der Plasmaquelle 5 wurde eine präklinische Studie durchgeführt, um ein sicheres therapeutisches Fenster für Behandlungen zu eruieren.

Zunächst wurden Effektivitätsuntersuchungen durchgeführt. Dabei zeigte sich, dass die Plasmaquelle 5 sehr effektiv Bakterien - auch multi-resistente Keime - und Pilze inaktiviert. Hier werden während einer Behandlungsdauer von nur 60 Sekunden bereits hohe Reduktionen um vier bis fünf Größenordnungen erreicht.

Weitere Untersuchungen zeigten, dass auch bakterielle Biofilme inaktiviert werden können. Innerhalb von 60 Sekunden Behandlungsdauer wurden Reduktionen um drei Größenordnungen erreicht. Eine vollständige Reduktion konnte nach einer Behandlungsdauer von 10 Minuten erzielt werden.

Weiterhin wurden Sicherheitsuntersuchungen durchgeführt, insbesondere Vitalitätsuntersuchungen an eukaryotischen Zellen (primäre Fibroblasten und Keratinozyten); Mutagenitätstests; Wundheilungsassays (zur Analyse der Proliferation der Zellen), und Untersuchungen an ex vivo-Haut (Histologie, Apoptose- bzw. Nekrose-Analyse).

Diese Untersuchungen zeigen, dass es sogar für den schlechtesten anzunehmenden Fall von einzelnen eukaryotischen Zellen bei Behandlungsdauern von bis zu 3 Minuten zu keiner Schädigung kommt. Die Mutagenitätsuntersuchungen zeigten für keine Plasmabehandlungsdauer (getestet bis 5 Minuten) eine Induktion von Mutationen, und auch die ex vivo-Hautuntersuchungen zeigten für keine Plasmabehandlungsdauer irgendwelche Schädigungen. Dies lässt auf ein noch deutlich größeres therapeutisches Fenster, als es hier spezifiziert wurde, schließen.

Mit dem hier beschriebenen Verfahren ist insbesondere eine initiale Überprüfung einer Elektrodenanordnung zur Erzeugung eines nicht-thermischen Plasmas möglich, was insbesondere eine Sicherheit des Betriebs der Elektrodenanordnung selbst und der jeweiligen Verwendung der Elektrodenanordnung deutlich erhöht.

**Patentansprüche**

1. Verfahren zum Prüfen einer Elektrodenanordnung (1) zur Erzeugung eines nicht-thermischen Plasmas, mit folgenden Schritten:

   - Bestimmen von wenigstens einem eine Plasma-Leistung der Elektrodenanordnung (1) charakterisierenden Leistungs-Parameter;
   - Vergleichen des wenigstens einen Leistungs-Parameters mit wenigstens einem vorbestimmten Soll-Parameterwert, und Erhalten eines Vergleichsergebnisses, und
   - Beurteilen einer Funktionsfähigkeit der Elektrodenanordnung (1) anhand des Vergleichsergebnisses,

   **dadurch gekennzeichnet, dass** der wenigstens eine vorbestimmte Soll-Parameterwert in Abhängigkeit von wenigstens einem Einsatzparameter der Elektrodenanordnung (1) in einem Kennfeld hinterlegt ist, aus dem er abhängig von dem wenigstens einen Einsatzparameter ausgelesen wird, wobei der wenigstens eine Einsatzparameter ausgewählt ist aus einer Gruppe, bestehend aus einer Umgebungstemperatur der Elektrodenanordnung (1) und einer relativen Luftfeuchte in einer Umgebung der Elektrodenanordnung (1).

2. Verfahren nach Anspruch 1, **dadurch gekennzeichnet, dass** wenigstens eine Aktion in Abhängigkeit von dem Vergleichsergebnis ausgewählt wird, wobei die Aktion vorzugsweise ausgewählt wird aus einer Gruppe, bestehend aus einer Ausgabe eines "In-Ordnung"-Signals, einer Ausgabe eines "Handlungsbedarf"-Signals, einer Ausgabe eines "Nicht-In-Ordnung"-Signals, einer Mitteilung einer momentanen Plasma-Leistung an einen Betreiber der Elektrodenanordnung (1), einer Anpassung einer Betriebsdauer der Elektrodenanordnung (1) an das Vergleichsergebnis, einem Beenden eines Betriebs der Elektrodenanordnung (1), und einem Fortsetzen des Betriebs der Elektrodenanordnung (1) ohne weitere Maßnahme.

3. Verfahren nach einem der vorhergehenden Ansprüche, **dadurch gekennzeichnet, dass** das Verfahren unmittelbar nach einer Inbetriebnahme der Elektrodenanordnung - insbesondere aber vor deren Anwendung - durchgeführt wird.

4. Verfahren nach einem der vorhergehenden Ansprüche, **dadurch gekennzeichnet, dass** der wenigstens eine Leistungs-Parameter an einer zu der Elektrodenanordnung (1) in Reihe geschalteten elektronischen Proxystruktur (104) erfasst wird.

5. Verfahren nach Anspruch 4, **dadurch gekennzeichnet, dass** als der wenigstens eine Leistungs-Parameter wenigstens ein Wert einer über der elektronischen Proxystruktur (104) abfallenden Proxyspannung zu einem bestimmten Phasenwinkel einer an die Elektrodenanordnung (1) angelegten Ansteuerspannung, insbesondere bei einem Nulldurchgang der Ansteuerspannung, gemessen wird.

6. Verfahren nach Anspruch 5, **dadurch gekennzeichnet, dass** als der wenigstens eine Leistungs-Parameter ein Mittelwert der Proxyspannung bei dem bestimmten Phasenwinkel der Ansteuerspannung, gemittelt über eine Mehrzahl, insbesondere eine Vielzahl, von Perioden der Ansteuerspannung ermittelt wird.

7. Verfahren nach einem der Ansprüche 4 bis 6, **dadurch gekennzeichnet, dass** als elektronische Proxystruktur (104) eine Kapazität (105), insbesondere ein Kondensator, verwendet wird.

8. Verfahren nach einem der vorhergehenden Ansprüche, **dadurch gekennzeichnet, dass** der wenigstens eine Leistungs-Parameter mit einem ersten, oberen Soll-Parameterwert und mit einem zweiten, unteren Soll-Parameterwert verglichen wird, wobei die wenigstens eine Aktion davon abhängig gewählt wird, ob der wenigstens eine Leistungs-Parameter in einen durch den ersten Soll-Parameterwert und den zweiten Soll-Parameterwert begrenzten Soll-Parameterbereich fällt.

9. Verfahren nach einem der vorhergehenden Ansprüche, **dadurch gekennzeichnet, dass** die Elektrodenanordnung (1) vor der Bestimmung des mindestens einen Leistungs-Parameters für eine vorbestimmte Zeitdauer betrieben wird.

10. Verfahren nach einem der vorhergehenden Ansprüche, **dadurch gekennzeichnet, dass** das Vergleichsergebnis und/oder der wenigstens eine Leistungs-Parameter für einen späteren Abruf in einer elektronischen Speichereinrichtung der Elektrodenanordnung (1) - insbesondere automatisiert, vorzugsweise mit wenigstens einem Metadatum - protokolliert wird/werden.

11. Verfahren nach einem der vorhergehenden Ansprüche, **dadurch gekennzeichnet, dass** eine Elektrodenanordnung (1) geprüft wird, die eingerichtet ist zur Erzeugung von Oberflächenmikroentladungen in Umgebungsluft.

12. Verfahren nach Anspruch 11, **dadurch gekennzeichnet, dass** eine Elektrodenanordnung (1) geprüft wird, die eine erste, vorzugsweise flächige Elektrode (3), eine zweite, vorzugsweise flächige Elektrode (5) und ein Dielektrikum (7) aufweist, wobei die erste Elektrode (3) und die zweite Elektrode (5) durch das Dielektrikum (7) voneinander beabstandet und jeweils in direktem mechanischen Kontakt zu dem Dielektrikum (7) angeordnet sind.

13. Verfahren nach einem der Ansprüche 11 oder 12, **dadurch gekennzeichnet, dass** an die erste Elektrode (3) eine Hochspannung, insbesondere eine Wechselspannung, angelegt wird, wobei die zweite Elektrode (5) auf Masse gelegt oder geerdet wird.

14. Plasmaquelle (100), mit einer Elektrodenanordnung (1) zum Erzeugen eines nicht-thermischen Plasmas, wobei die Elektrodenanordnung (1)

- eine erste Elektrode (3),
- eine zweite Elektrode (5), und
- ein Dielektrikum (7) aufweist, durch das die erste Elektrode (3) und die zweite Elektrode (5) voneinander beabstandet sind, wobei
- die erste Elektrode (3) an einer ersten Seite (9) des Dielektrikums (7) angeordnet ist, wobei
- die zweite Elektrode (5) an einer zweiten, der ersten Seite (9) gegenüberliegenden Seite (11) des Dielektrikums (7) angeordnet ist, und mit
- einer Steuereinrichtung (101), die eingerichtet ist zur Ansteuerung der Elektrodenanordnung, wobei
- die Steuereinrichtung (101) eingerichtet ist zur Durchführung eines Verfahrens nach einem der Ansprüche 1 bis 13.

15. Plasmaquelle (100) nach Anspruch 14, **dadurch gekennzeichnet, dass** die Steuereinrichtung (101) eine elektronische Proxystruktur (104) aufweist, die in Reihe mit der Elektrodenanordnung (1) schaltbar oder geschaltet ist, wobei die Steuereinrichtung (101) eingerichtet ist, um den wenigstens einen Leistungs-Parameter an der zu der Elektrodenanordnung (1) in Reihe geschalteten elektronischen Proxystruktur (104) zu erfassen.

16. Plasmaquelle (100) nach Anspruch 15, **dadurch gekennzeichnet, dass** die elektronische Proxystruktur (104) eine Kapazität (105), insbesondere einen Kondensator oder eine Kondensator-Anordnung, ist.

17. Plasmaquelle (100) nach einem der Ansprüche 14 bis 16, **dadurch gekennzeichnet, dass** die zweite Elektrode (5) eine periodische Struktur aufweist.

18. Plasmaquelle (100) nach einem der vorhergehenden Ansprüche 14 bis 17, **dadurch gekennzeichnet, dass**

a) die zweite Elektrode (5) wenigstens ein Strukturelement (15) mit wenigstens einer von Kanten (17) begrenzten Ausnehmung (19) aufweist, wobei die die Ausnehmung (19) begrenzenden Kanten (17) innerhalb jeder Ausnehmung (19) einen Abstand von mindestens 0,5 mm bis höchstens 10 mm, vorzugsweise von mindestens 1 mm bis höchstens 8 mm, vorzugsweise von mindestens 2 mm bis höchstens 7 mm, vorzugsweise von 5 mm, zueinander aufweisen, und/oder dass
b) die zweite Elektrode (5) eine Mehrzahl von Strukturelementen (15) aufweist, wobei die einzelnen Strukturelemente (15) einen Abstand von mindestens 0,5 mm bis höchstens 10 mm, vorzugsweise von mindestens 1 mm bis höchstens 8 mm, vorzugsweise von mindestens 2 mm bis höchstens 7 mm, vorzugsweise von 5 mm, zueinander aufweisen, und/oder dass
c) die zweite Elektrode (5) linear, zick-zack-förmig, gerade, bogenförmig, wellig, spiralig, kammartig oder mäanderförmig ausgebildet ist.

19. Plasmaquelle (100) nach einem der vorhergehenden Ansprüche 14 bis 18, **dadurch gekennzeichnet, dass**

a) das Dielektrikum (7) und die zweite Elektrode (5) die erste Elektrode (3) allseitig - senkrecht zu einer Stapelrichtung der Elektrodenanordnung (1) gesehen - überragen, oder dass
b) das Dielektrikum (7) und die erste Elektrode (3) die zweite Elektrode (5) allseitig - senkrecht zu einer Stapelrichtung der Elektrodenanordnung (1) gesehen - überragen, oder dass
c) das Dielektrikum (7) die erste Elektrode (3) und die zweite Elektrode (5) allseitig - senkrecht zu einer Stapelrichtung der Elektrodenanordnung (1) gesehen - überragt.

20. Plasmaquelle (100) nach einem der vorhergehenden Ansprüche 14 bis 19, **dadurch gekennzeichnet, dass** die erste Elektrode (3) mit einer Isolierschicht (13) beschichtet und/oder mit einer Vergussmasse vergossen ist.

**Claims**

1. Method for testing an electrode arrangement (1) for generating a non-thermal plasma, comprising the following steps:

- determining at least one power parameter characterising a plasma power of the electrode arrangement (1);
- comparing the at least one power parameter with at least one predetermined target parameter value, and obtaining a comparison result, and
- assessing the functionality of the electrode arrangement (1) on the basis of the comparison result,

**EP 3 808 159 B1**

**characterised in that** at least one predetermined target parameter value is stored with a dependence on at least one operational parameter of the electrode arrangement (1) in a characteristic map, from which it is read out depending on the at least one operational parameter, wherein the at least one operational parameter is selected from a group consisting of an ambient temperature of the electrode arrangement (1) and a relative humidity in an environment of the electrode arrangement (1).

2. Method according to claim 1, **characterised in that** at least one action is selected depending on the comparison result, wherein the action is preferably selected from a group consisting of outputting an "OK" signal, outputting an "action required" signal, outputting a "not OK" signal, a notification of a current plasma power to an operator of the electrode arrangement (1), an adjustment of an operating time of the electrode arrangement (1) to the comparison result, a termination of an operation of the electrode arrangement (1), and a continuation of the operation of the electrode arrangement (1) without further action.

3. Method according to any of the preceding claims, **characterised in that** the method is carried out immediately after the electrode arrangement has been put into operation - but in particular before it is used.

4. Method according to any of the preceding claims, **characterised in that** the at least one power parameter is detected at an electronic proxy structure (104) connected in series with the electrode arrangement (1).

5. Method according to claim 4, **characterised in that** at least one value of a proxy voltage dropping across the electronic proxy structure (104) at a specific phase angle of a control voltage applied to the electrode arrangement (1), in particular at a zero crossing of the control voltage, is measured as the at least one power parameter.

6. Method according to claim 5, **characterised in that** an average value of the proxy voltage at the specific phase angle of the control voltage, averaged over a plurality, in particular a multiplicity, of periods of the control voltage is determined as the at least one power parameter.

7. Method according to any of claims 4 to 6, **characterised in that** a capacitance (105), in particular a capacitor, is used as the electronic proxy structure (104).

8. Method according to any of the preceding claims, **characterised in that** the at least one power parameter is compared with a first, upper target parameter value and with a second, lower target parameter value, wherein the at least one action is selected depending on whether the at least one power parameter falls within a target parameter range limited by the first target parameter value and the second target parameter value.

9. Method according to any of the preceding claims, **characterised in that** the electrode arrangement (1) is operated for a predetermined period of time before the at least one power parameter is determined.

10. Method according to any of the preceding claims, **characterised in that** the comparison result and/or the at least one power parameter is/are logged in an electronic memory device of the electrode arrangement (1) for later retrieval, in particular in an automated manner, preferably with at least one metadata item.

11. Method according to any of the preceding claims, **characterised in that** an electrode arrangement (1) is tested which is configured to generate surface micro-discharges in ambient air.

12. Method according to claim 11, **characterised in that** an electrode arrangement (1) is tested which has a first, preferably planar electrode (3), a second, preferably planar electrode (5), and a dielectric (7), wherein the first electrode (3) and the second electrode (5) are spaced apart from each other by the dielectric (7) and are each arranged in direct mechanical contact with the dielectric (7).

13. Method according to any of claims 11 or 12, **characterised in that** a high voltage, in particular an alternating voltage, is applied to the first electrode (3), wherein the second electrode (5) is connected to ground or earthed.

14. Plasma source (100) with an electrode arrangement (1) for generating a non-thermal plasma, wherein the electrode arrangement (1) comprises

    - a first electrode (3),
    - a second electrode (5), and

23

- a dielectric (7), by means of which the first electrode (3) and the second electrode (5) are spaced apart from each other, wherein
- the first electrode (3) is arranged on a first side (9) of the dielectric (7), wherein
- the second electrode (5) is arranged on a second side (11) of the dielectric (7) opposite the first side (9), and with
- a control device (101) which is configured to control the electrode arrangement, wherein
- the control device (101) is configured to carry out a method according to any of claims 1 to 13.

15. Plasma source (100) according to claim 14, **characterised in that** the control device (101) has an electronic proxy structure (104) which is connectable or is connected in series with the electrode arrangement (1), wherein the control device (101) is configured to detect the at least one power parameter at the electronic proxy structure (104) connected in series with the electrode arrangement (1).

16. Plasma source (100) according to claim 15, **characterised in that** the electronic proxy structure (104) is a capacitance (105), in particular a capacitor or a capacitor arrangement.

17. Plasma source (100) according to any of claims 14 to 16, **characterised in that** the second electrode (5) has a periodic structure.

18. Plasma source (100) according to any of the preceding claims 14 to 17, **characterised in that**

a) the second electrode (5) has at least one structural element (15) with at least one recess (19) delimited by edges (17), wherein the edges (17) delimiting the recess (19) inside each recess (19) are at a distance to each other of at least 0.5 mm to at most 10 mm, preferably at least 1 mm to at most 8 mm, preferably at least 2 mm to at most 7 mm, preferably 5 mm, and/or that
b) the second electrode (5) has a plurality of structural elements (15), wherein the individual structural elements (15) are spaced apart from each other by at least 0.5 mm to at most 10 mm, preferably at least 1 mm to at most 8 mm, preferably at least 2 mm to at most 7 mm, preferably by 5 mm, and/or **in that**
c) the second electrode (5) is designed to be linear, zigzag-shaped, straight, curved, wavy, spiral-shaped, comb-like or meander-shaped.

19. Plasma source (100) according to any of the preceding claims 14 to 18, **characterised in that**

a) the dielectric (7) and the second electrode (5) protrude beyond the first electrode (3) on all sides - viewed perpendicular to a stacking direction of the electrode arrangement (1) - or that
b) the dielectric (7) and the first electrode (3) protrude beyond the second electrode (5) on all sides - viewed perpendicular to a stacking direction of the electrode arrangement (1) - or that
c) the dielectric (7) protrudes beyond the first electrode (3) and the second electrode (5) on all sides - viewed perpendicular to a stacking direction of the electrode arrangement (1).

20. Plasma source (100) according to any of the preceding claims 14 to 19, **characterised in that** the first electrode (3) is coated with an insulating layer (13) and/or encapsulated with a casting compound.

**Revendications**

1. Procédé de contrôle d'un ensemble d'électrodes (1) pour la production d'un plasma non thermique, comprenant les étapes suivantes :

- détermination d'au moins un paramètre de puissance caractérisant une puissance plasma de l'ensemble d'électrodes (1) ;
- comparaison de l'au moins un paramètre de puissance avec au moins une valeur de paramètre de consigne prédéterminée, et obtention d'un résultat de comparaison, et
- évaluation d'une capacité de fonctionnement de l'ensemble d'électrodes (1) à l'aide du résultat de comparaison, **caractérisé en ce que** l'au moins une valeur de paramètre de consigne prédéterminée est enregistrée dans un champ caractéristique en fonction d'au moins un paramètre d'utilisation de l'ensemble d'électrodes (1), à partir duquel elle est lue en fonction de l'au moins un paramètre d'utilisation,
dans lequel l'au moins un paramètre d'utilisation est sélectionné parmi un groupe comprenant une température ambiante de l'ensemble d'électrodes (1) et une humidité relative de l'air dans un environnement de l'ensemble d'électrodes (1).

2. Procédé selon la revendication 1, **caractérisé en ce qu'**au moins une action est sélectionnée en fonction du résultat de comparaison, dans lequel l'action est de préférence sélectionnée dans un groupe comprenant l'émission d'un signal « conforme », l'émission d'un signal « action requise », l'émission d'un signal « non conforme », un message d'une puissance plasma momentanée à un exploitant de l'ensemble d'électrodes (1), une adaptation d'une durée de fonctionnement de l'ensemble d'électrodes (1) au résultat de comparaison, une interruption du fonctionnement de l'ensemble d'électrodes (1) et une poursuite du fonctionnement de l'ensemble d'électrodes (1) sans autre mesure.

3. Procédé selon l'une des revendications précédentes, **caractérisé en ce que** le procédé est effectué immédiatement après la mise en service de l'ensemble d'électrodes, en particulier avant son utilisation.

4. Procédé selon l'une des revendications précédentes, **caractérisé en ce que** l'au moins un paramètre de puissance est saisi au niveau d'une structure électronique proxy (104) branchée en série à l'ensemble d'électrodes (1).

5. Procédé selon la revendication 4, **caractérisé en ce que,** en tant que l'au moins un paramètre de puissance, au moins une valeur d'une tension proxy chutant sur la structure électronique proxy (104) est mesurée à un angle de phase déterminé d'une tension de commande appliquée à l'ensemble d'électrodes (1), en particulier lors d'un passage par zéro de la tension de commande.

6. Procédé selon la revendication 5, **caractérisé en ce qu'**une valeur moyenne de la tension proxy est calculée en tant que l'au moins un paramètre de puissance à l'angle de phase déterminé de la tension de commande, calculée sur une pluralité, en particulier une multitude, de périodes de la tension de commande.

7. Procédé selon l'une des revendications 4 à 6, **caractérisé en ce qu'**une capacité (105), en particulier un condensateur, est utilisée comme structure électronique proxy (104).

8. Procédé selon l'une des revendications précédentes, **caractérisé en ce que** l'au moins un paramètre de puissance est comparé à une première valeur de paramètre de consigne supérieure et à une seconde valeur de paramètre de consigne inférieure, dans lequel l'au moins une action est choisie en fonction du fait que l'au moins un paramètre de puissance se trouve dans une plage de paramètres de consigne limitée par la première valeur de paramètre de consigne et par la seconde valeur de paramètre de consigne.

9. Procédé selon l'une des revendications précédentes, **caractérisé en ce que** l'ensemble d'électrodes (1) est exploité pendant une durée prédéterminée avant la détermination de l'au moins un paramètre de puissance.

10. Procédé selon l'une des revendications précédentes, **caractérisé en ce que** le résultat de comparaison et/ou l'au moins un paramètre de puissance sont enregistrés dans une mémoire électronique de l'ensemble d'électrodes (1) en vue d'une consultation ultérieure, en particulier de manière automatisée, de préférence avec au moins une métadonnée.

11. Procédé selon l'une des revendications précédentes, **caractérisé en ce qu'**un ensemble d'électrodes (1) est contrôlé, lequel est conçu pour produire des microdécharges superficielles dans l'air ambiant.

12. Procédé selon la revendication 11, **caractérisé en ce qu'**un ensemble d'électrodes (1) est contrôlé, lequel comprend une première électrode (3) de préférence plane, une seconde électrode (5) de préférence plane et un diélectrique (7), dans lequel la première électrode (3) et la seconde électrode (5) sont espacées l'une de l'autre par le diélectrique (7) et sont chacune disposées en contact mécanique direct avec le diélectrique (7).

13. Procédé selon l'une des revendications 11 ou 12, **caractérisé en ce qu'**une haute tension, en particulier une tension alternative, est appliquée à la première électrode (3), dans lequel la seconde électrode (5) est mise à la masse ou mise à la terre.

14. Source de plasma (100) dotée d'un ensemble d'électrodes (1) pour la production d'un plasma non thermique, dans lequel l'ensemble d'électrodes (1) comprend

- une première électrode (3),
- une seconde électrode (5), et

- un diélectrique (7), qui sépare la première électrode (3) et la seconde électrode (5) l'une de l'autre, dans lequel
- la première électrode (3) est disposée sur un premier côté (9) du diélectrique (7), dans lequel
- la seconde électrode (5) est disposée sur un second côté (11) du diélectrique (7) opposé au premier côté (9), et avec
- un dispositif de commande (101), qui est conçu pour commander l'ensemble d'électrodes, dans lequel
- le dispositif de commande (101) est conçu pour exécuter un procédé selon l'une des revendications 1 à 13.

15. Source de plasma (100) selon la revendication 14, **caractérisé en ce que** le dispositif de commande (101) comprend une structure électronique proxy (104) qui peut être branchée ou est branchée en série avec l'ensemble d'électrodes (1), dans lequel le dispositif de commande (101) est conçu pour saisir l'au moins un paramètre de puissance au niveau de la structure électronique proxy (104) branchée en série à l'ensemble d'électrodes (1).

16. Source de plasma (100) selon la revendication 15, **caractérisé en ce que** la structure électronique proxy (104) est une capacité (105), en particulier un condensateur ou un ensemble de condensateurs.

17. Source de plasma (100) selon l'une des revendications 14 à 16, **caractérisé en ce que** la seconde électrode (5) présente une structure périodique.

18. Source de plasma (100) selon l'une des revendications 14 à 17 précédentes, **caractérisé en ce que**

a) la seconde électrode (5) comporte au moins un élément structurel (15) avec au moins un évidement (19) délimité par des arêtes (17), dans lequel les arêtes (17) qui délimitent l'évidement (19) présentent, à l'intérieur de chaque évidement (19), un écart d'au moins 0,5 mm à 10 mm au plus, de préférence d'au moins 1 mm à 8 mm au plus, de préférence d'au moins 2 mm à 7 mm au plus, de préférence de 5 mm, les uns par rapport aux autres, et/ou
b) la seconde électrode (5) comporte une pluralité d'éléments structurels (15), dans lequel les différents éléments structurels (15) présentent un écart d'au moins 0,5 mm à 10 mm au plus, de préférence d'au moins 1 mm à 8 mm au plus, de préférence d'au moins 2 mm à 7 mm au plus, de préférence de 5 mm, les uns par rapport aux autres, et/ou
c) la seconde électrode (5) est conçue de manière linéaire, en zigzag, de manière rectiligne, courbée, ondulée, en spirale, en peigne ou en méandres.

19. Source de plasma (100) selon l'une des revendications 14 à 18, **caractérisé en ce que**

a) le diélectrique (7) et la seconde électrode (5) dépassent de la première électrode (3) de tous les côtés, vu perpendiculairement à une direction d'empilement de l'ensemble d'électrodes (1), ou
b) le diélectrique (7) et la première électrode (3) dépassent de tous les côtés de la seconde électrode (5), vu perpendiculairement à une direction d'empilement de l'ensemble d'électrodes (1), ou
c) le diélectrique (7) dépasse de tous les côtés de la première électrode (3) et de la seconde électrode (5), vu perpendiculairement à une direction d'empilement de l'ensemble d'électrodes (1).

20. Source de plasma (100) selon l'une des revendications 14 à 19, **caractérisé en ce que** la première électrode (3) est recouverte d'une couche isolante (13) et/ou moulée avec une masse de scellement.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1693014 A1